# EUROPEAN PATENT SPECIFICATION

(11) **EP 2 396 072 B1**
(45) Date of publication and mention of the grant of the patent: **06.03.2013**
(21) Application number: 10701956.4
(22) Date of filing: 14.01.2010
(51) Int. Cl.: A61N 1/05, A61B 17/34, A61M 25/00, A61M 25/01

(54) **STIMULATION LEAD DELIVERY SYSTEM**
ABGABESYSTEM FÜR STIMULATIONSLEITUNGEN
SYSTEMES DE POSE D'ELECTRODES DE STIMULATION

(30) Priority: 16.10.2009 US 252270 P; 14.01.2009 US 144690 P
(43) Date of publication of application: 21.12.2011
(73) Proprietor: Spinal Modulation Inc., Menlo Park, CA 94025 (US)
(72) Inventor: LINKER, Fred, I., Menlo Park, CA 94025 (US); BROUNSTEIN, Daniel, M., Menlo Park, CA 94025 (US); BURDULIS, Albert, G., Menlo Park, CA 94025 (US); JOHNSON, Eric, T., Menlo Park, CA 94025 (US); BURKE, Phillip, C., Menlo Park, CA 94025 (US); VANDENBRINK, Evan, S., Menlo Park, CA 94025 (US); GRIGSBY, Eric, J., Menlo Park, CA 94025 (US); TAN, Henry, L.s., Menlo Park, CA 94025 (US)
(74) Representative: Dee, Ian Mark
(86) International application number: PCT/US2010/021041
(87) International publication number: WO 2010/083308

(56) References cited:
- US-A1- 2003 144 657
- US-A1- 2005 251 237
- US-A1- 2008 009 927
- US-A1- 2008 103 579
- US-A1- 2008 140 153
- US-A1- 2008 140 169
- US-A1- 2008 183 257
- US-A1- 2008 188 916

## Description

### BACKGROUND OF THE INVENTION

The application of specific electrical energy to the spinal cord for the purpose of managing pain has been actively practiced since the 1960s. It is known that application of an electrical field to spinal nervous tissue can effectively mask certain types of pain transmitted from regions of the body associated with the stimulated nervous tissue. Such masking is known as paresthesia, a subjective sensation of numbness or tingling in the afflicted bodily regions. Such electrical stimulation of the spinal cord, once known as dorsal column stimulation, is now referred to as spinal cord stimulation or SCS.

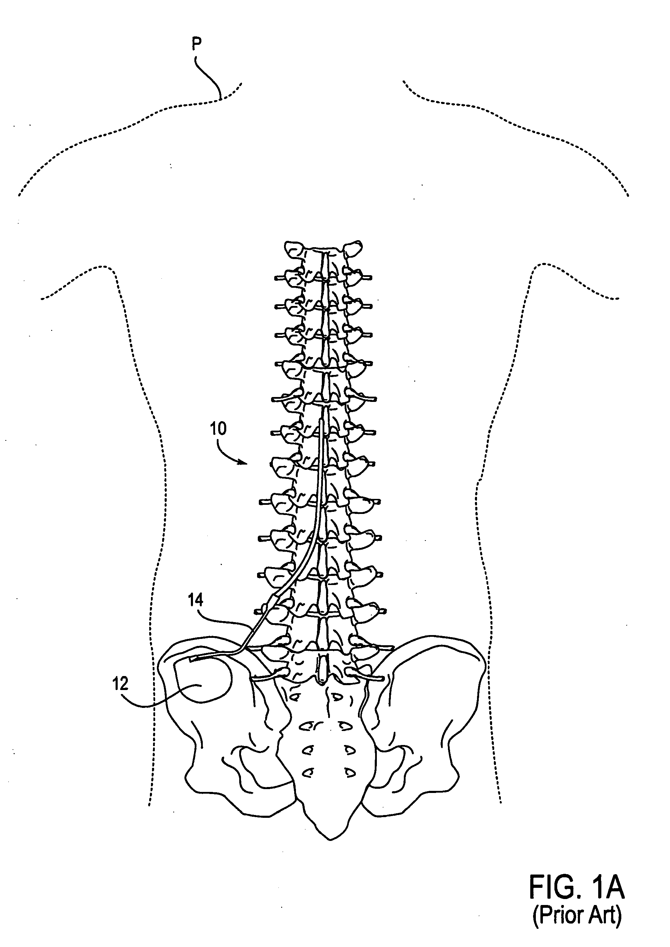

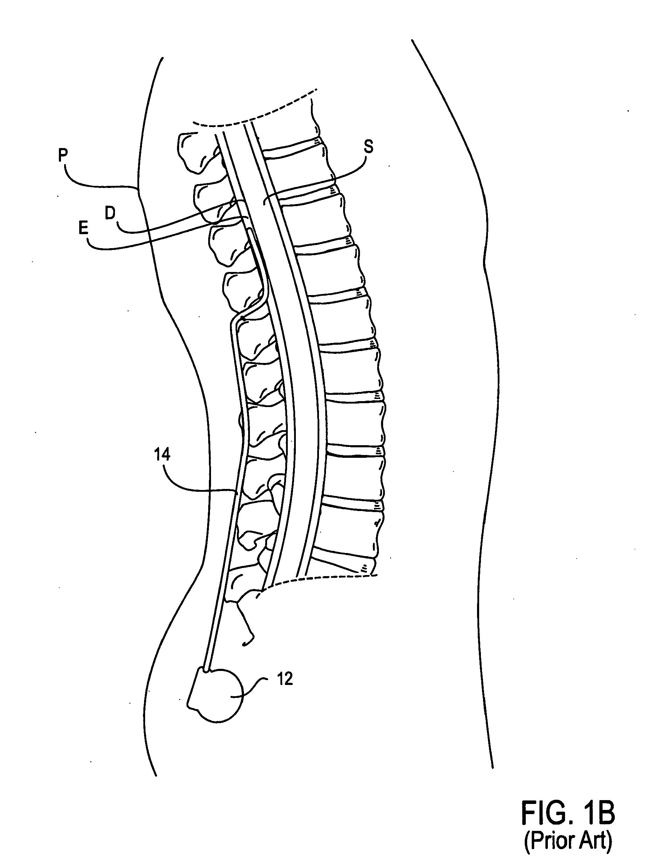

Figs. 1A-1B illustrate conventional placement of an SCS system 10. Conventional SCS systems typically include an implantable power source or implantable pulse generator (IPG) 12 and an implantable lead 14. Such IPGs 12 are similar in size and weight to pacemakers and are typically implanted in the buttocks of a patient P, as shown, or in the abdominal wall, chest wall, or under the arm. Using fluoroscopy, the lead 14 is implanted into the epidural space E of the spinal column and positioned against the dura layer D of the spinal cord S, as illustrated in Fig. 1B.

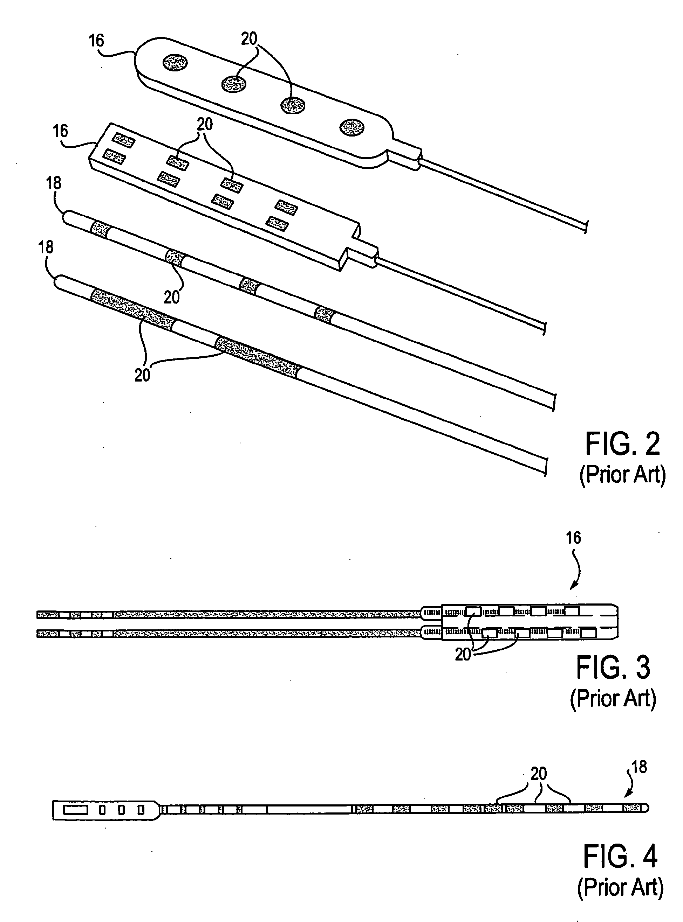

Fig. 2 illustrates example conventional paddle leads 16 and percutaneous leads 18. Paddle leads 16 typically have the form of a slab of silicon rubber having one or more electrodes 20 on its surface. Example dimensions of a paddle lead 16 are illustrated in Fig. 3. Percutaneous leads 18 typically have the form of a tube or rod having one or more electrodes 20 extending therearound. Example dimensions of a percutaneous lead 18 are illustrated in Fig. 4.

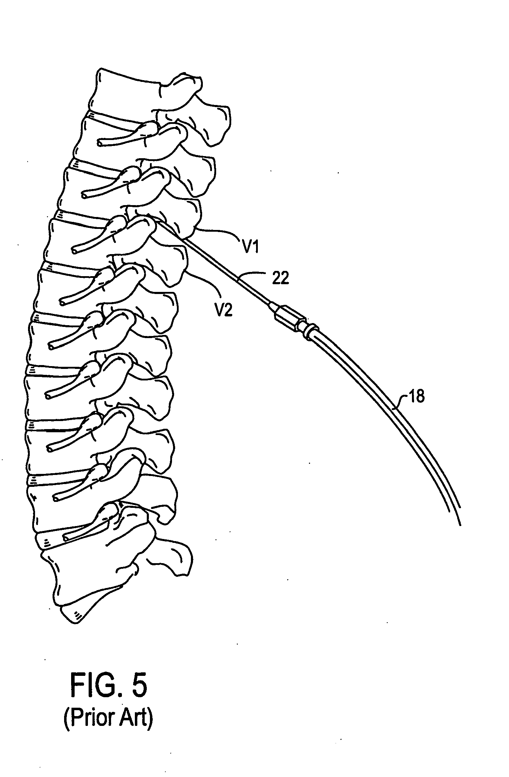

Paddle leads 16 and percutaneous leads 18 are positioned within the epidural space E of the spinal column by different methods due to their size and shape. Percutaneously leads 18 are positioned with the use of an epidural needle. Referring to Fig. 5, an epidural needle 22 is inserted through the skin (not shown) and advanced between adjacent vertebrae V1, V2 so that it penetrates the epidural space. Thus, a conduit is formed from outside of the body to the epidural space. The lead 18 is then advanced through the needle 22 and into the epidural space. The lead 18 is typically advanced in an antegrade fashion up the midline of the spinal column until it reaches the area of the spinal cord that, when electrically stimulated, produces a tingling sensation (paresthesia) that covers the patient's painful area. To locate this area, the lead is moved and/or turned on and off while the patient provides feedback about stimulation coverage. Often, inadequate stimulation is obtained and the lead may be repositioned multiple times before adequate coverage is received. Because the patient participates in this operation and directs the operator to the correct area of the spinal cord, the procedure is performed under monitored anesthesia care.

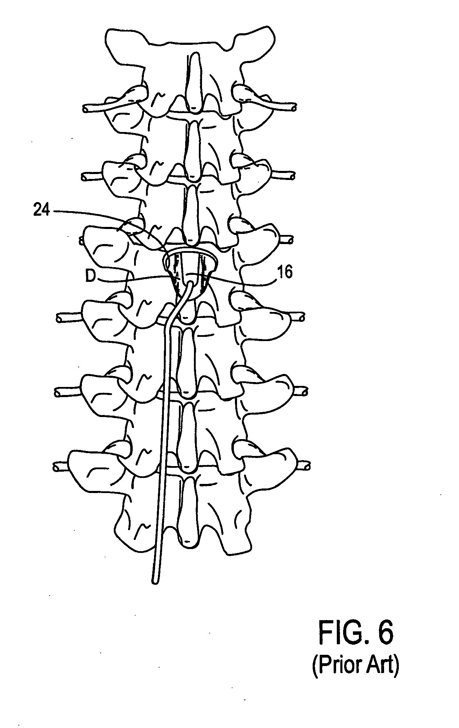

Conventional paddle leads 16 are too large to fit through an epidural needle. Therefore, implantation of paddle leads 16 typically involves a mini laminotomy. A laminotomy is a neurosurgical procedure that removes part of a lamina of the vertebral arch. An incision is typically made slightly below the spinal cord segment to be stimulated. The laminotomy creates an opening 24 in the bone large enough to pass one or more paddle leads 16 through. Fig. 6 illustrates a mini laminotomy with a paddle lead 16 inserted therethrough so that the stimulating portion of the lead 16 resides against the dura layer D of the spinal cord S. The target area for stimulation usually has been located before this procedure during a spinal cord stimulation trial with percutaneous leads 18.

As with any surgery, surgical placement of stimulation leads is a serious procedure and should be treated as such. A variety of complications may result, including complications with the anesthesia medication, deep vein thrombosis (DVT), nerve damage, and infection, to name a few. Thus, less invasive procedures are desired. Such procedures should be effective in treating pain while minimizing complications, cost and debilitation. At least some of these objectives will be met by the present invention.
US 2003/0144657 discloses a catheter delivery system having inner and outer catheters, both of which have curved distal ends. The catheter assembly can include at least one electrode at the distal end of either the inner or outer catheter. A pacing lead can also be inserted through the inner or outer catheter.

US 2005/0251237 discloses a system having a lead carrying an electrode and an insertion tube.

US 2008/0009927 discloses a device comprising an electrical stimulation lead and an introducer needle, which can have a bent distal tip. The document also discloses a device having a sheath that is placed within an introducer needle and a stimulation lead that is placed within the sheath.

US 2008/0103579 discloses a system comprising a medical lead and an outer sheath for enclosing the lead. The lead comprises an electrode and an adhesive element.

US 2008/0183257 discloses a system having a lead carrying an electrode and a retention feature to assist with anchoring the lead.

US 2008/0140169 discloses a system having a lead carrying an electrode and an implantable pulse generator.

US 2008/0140153 discloses a lead comprising an electrode.

### BRIEF SUMMARY OF THE INVENTION

According to the present invention there is provided a system for accessing a nerve root according to claim 1.

The present invention provides a system for accessing and treating anatomies associated with a variety of conditions while minimizing possible complications and side effects. This is achieved by directly neuromodulating a target anatomy associated with the condition while minimizing or excluding undesired neuromodulation of other anatomies. Typically, this involves stimulating portions of neural tissue of the central nervous system, wherein the central nervous system includes the spinal cord and the pairs of nerves along the spinal cord which are known as spinal nerves. In particular, some embodiments of the present invention are used to selectively stimulate portions of the spinal nerves, particularly one or more dorsal root ganglions (DRGs), to treat chronic pain while causing minimal deleterious side effects such as undesired motor responses. Such stimulation is achieved with the use of a lead having at least one electrode thereon. The lead is advanced through the patient anatomy so that the at least one electrode is positioned on, near or about the target anatomy.

The present system includes a lead comprising a shaft having at least one electrode disposed thereon, and a sheath having a curved distal end, wherein the sheath is configured to extend over the shaft of the lead causing the lead to bend. In an embodiment, the sheath has an outer diameter which allows advancement through an introducing needle into an epidural space of a spinal column and a stiffness which allows advancement along the epidural space to a position wherein the curved distal end of the sheath directs the lead toward the spinal nerve, and wherein withdrawal of the sheath positions the lead near the spinal nerve.

In some embodiments, the introducing needle has an inner diameter of less than or equal to approximately 1.7 mm (0.067 inches). Typically, a 14 gauge needle has an inner diameter of 0.067 inches. In other embodiments, the sheath has a minimum stiffness of approximately 4.48 kPa (0.65 lbs·in2). The sheath may be comprised of a variety of materials, such as polyimide or polyetheretherketone. In some embodiments, the lead has a shaped distal tip, wherein the sheath is configured to extend over the shaft of the lead until a portion of the distal end abuts the shaped distal tip of the lead resisting further advancement of the sheath. Optionally, the distal tip of the lead provides an atraumatic cover for the distal end of the sheath.

The lead includes a stylet lumen extending at least partially therethrough, wherein the system includes a stylet configured to be positioned within the stylet lumen of the lead so that advancement of the stylet and withdrawal of the sheath positions the lead near the spinal nerve. The style has a curved distal end, wherein the positioning the curved distal end of the sheath over the lead bends the lead along a first curvature toward the spinal nerve and wherein advancement of the lead and stylet therein beyond the sheath bends the lead along a second curvature so that the lead extends from the spinal column along a nerve root angulation. In some instances, the nerve root angulation is equal to or less than 90 degrees. And in some instances, the nerve root angulation is equal to or less than 45 degrees. In some embodiments, the distal end of the stylet is curved having a primary curve and a secondary curve.

In some embodiments, the system further comprises an additional sheath having a distal end, wherein the additional sheath is configured to pass within the sheath so that its distal end extends beyond the curved distal end of the sheath. The distal end of the additional sheath may be curved so that positioning the curved distal end of the sheath over the lead bends the lead along a first curvature toward the spinal nerve and wherein advancement of the curved distal end of the additional sheath beyond the curved distal end of the sheath bends the lead along a second curvature toward a nerve root angulation. Or the distal end of the additional sheath may be substantially straight so that positioning the curved distal end of the sheath over the lead bends the lead along a first curvature toward the spinal nerve and wherein advancement of the curved distal end of the additional sheath beyond the curved distal end of the sheath directs the lead in a substantially straight direction toward the spinal nerve.

In some embodiments, the distal end of the sheath is curved having an angle in the range of approximately 80 to 165 degrees. In other embodiments, the distal end of the stylet is curved having a primary curve and a secondary curve. Optionally, the primary curve may have an arch shape of approximately 180 degrees. Optionally, the secondary curve may be proximal and adjacent to the primary curve. Optionally, the secondary curve may have a larger radius of curvature than the primary curve. In some embodiments, the lead has a closed-end distal tip having a shape which resists advancement of the sheath over the distal tip. Optionally, the shape may comprise a ball shape. In some embodiments, the lead is sized to fill an inner diameter of the sheath so as to resist kinking of the sheath. In other embodiments, the shaped distal tip of the lead provides an atraumatic cover for the distal end of the sheath.

In some embodiments, the sheath is comprised of a thermoset material. In some embodiments, the sheath is comprised of a unidurometer material. Optionally, the sheath may be at least partially radiopaque, such as loaded with radiopaque material. Or, the sheath may include at least one radiopaque marker.

In an embodiment, and second sheaths together have a stiffness which allows advancement along the epidural space to a position wherein the distal ends of the first and second sheaths direct the lead toward the spinal nerve.

In some embodiments, advancement of the lead and stylet therein beyond the second sheath bends the lead along a second curvature so that the lead extends from the spinal column along a nerve root angulation. Optionally, the system further comprises a control hub connectable with a proximal end of the first sheath and a proximal end of the second sheath, wherein manipulation of the control hub moves the first or second sheath in relation to each other. In some embodiments, the control hub includes a limiter, wherein the limiter limits the movement of the first or second sheath in relation to each other. In some embodiments, manipulation of the control hub is achievable with the use of one hand.

In an embodiment, the stimulation lead comprises a shaft comprising a tube having a distal end and a proximal end, a stylet tube disposed within the shaft, at least one electrode disposed near the distal end of the shaft, and at least one conductor cable extending from the at least one electrode toward the proximal end of the shaft. The stylet tube is fixedly coupled to the shaft at a first location near the distal end and at a second location proximal to the first location allowing for movement of the stylet tube within the shaft therebetween.

In some embodiments, the at least one conductor cable is disposed between the stylet tube and shaft, wherein the at least one conductor cable is fixedly coupled to the shaft near the proximal end and another location allowing for movement within the shaft therebetween. In some embodiments, the lead further comprises a tensile element fixedly coupled to the shaft in at least one location along the shaft. Optionally, the tensile element may have freedom of movement within the shaft outside of the at least one location. In some embodiments, the tensile element has multiple diameters. For example, the tensile element may have a larger diameter near its proximal end and neck down toward its distal end. In some embodiments, the stylet tube has a lubricious inner surface. Optionally, the stylet tube may be comprised of polyimide.

In some embodiments, at least a portion of the distal end of the shaft is configured to extend at least 180 degrees along the perimeter of a half circle, wherein the half circle has a radius of 6·35 mm (0·25 inches).

Other objects and advantages of the present invention will become apparent from the detailed description to follow, together with the accompanying drawings.

### BRIEF DESCRIPTION OF THE DRAWINGS

Figs. 1A, 1B, 2, 3, 4, 5, 6 illustrate prior art.

Fig. 7 illustrates a lead advanced through a nerve root sleeve angulation so that at least one of its electrodes is positioned within a clinically effective distance of a target DRG.

Figs. 8A, 8B, 8C, 8D illustrate an embodiment of a lead and delivery system, including a sheath, stylet and introducing needle of the present invention.

Fig. 9 illustrates an embodiment of a sheath advanced over a shaft of a lead with internal stylet forming a first curvature.

Fig. 10 illustrates the lead with internal stylet of Fig. 9 extending beyond the sheath forming a second curvature.

Fig. 11 illustrates a method of accessing an epidural space with the use of an introducing needle.

Fig. 12 illustrates a method of attaching a syringe to the needle of Fig. 11.

Fig. 13 illustrates a method of inserting a stylet, lead and sheath of the present invention through the needle of Fig. 11 into the epidural space.

Fig. 14 illustrates the distal end of the needle passed through the ligamentum flavum into the epidural space and the assembled sheath/lead/stylet of Fig. 13 emerging therefrom.

Fig. 15 illustrates advancing the assembled sheath/lead/stylet of Fig. 13 within the epidural space toward a target DRG.

Fig. 16 illustrates the precurvature of the sheath directing the lead laterally outwardly.

Fig. 17 illustrates the lead extending beyond the distal end of the sheath of Fig. 16.

Fig. 18 illustrates a method of using the needle of Fig. 11 to position an additional lead within the epidural space.

Fig. 19 illustrates an additional assembled sheath/lead/stylet advanced within the epidural space toward another or second target DRG.

Fig. 20 illustrates the precurvature of the sheath of Fig. 19 directing the lead laterally outwardly.

Fig. 21 illustrates the lead advanced beyond the distal end of the sheath of Fig. 20.

Fig. 22 illustrates a plurality of leads positioned within the epidural space, each lead stimulating a different DRG.

Fig. 23A illustrates an embodiment of a sheath of the present system.

Fig. 23B illustrates an embodiment of a hub having a locking cap and injection port.

Figs. 24A, 24B, 24C, 24D, 24E illustrate an embodiment of a lead of the present system.

Fig. 24F illustrates an embodiment lead of the present system comprising a multi-lumen tubing.

Figs. 25, 26A-26B illustrate embodiments of a stylet of the present system.

Fig. 27 illustrates an embodiment of a system of the present invention having multiple sheaths.

Fig. 28 illustrates the system of Fig. 27 positioned within the epidural space.

Figs. 29A, 29B, 29C illustrate a perspective view, a side view and a front view, respectively, of an embodiment of a control hub.

Fig. 30 illustrates a conventional stimulation system used to stimulate tissues or organs within the body.

Fig. 31 illustrates an embodiment of a strain relief support of the present system.

Fig. 32 illustrates a cross-section of the strain relief support, including the support member and the hub.

Figs. 33-36 illustrate insertion of the support member into the proximal end of a lead and detachment of the hub.

Fig. 37 illustrates the proximal end of the lead inserted into the connection port of the IPG.

### DETAILED DESCRIPTION OF THE INVENTION

The present invention provides systems for accessing and treating anatomies associated with a variety of conditions, particularly conditions that are associated with or influenced by the nervous system. Examples of such conditions include pain, itching, Parkinson's Disease, Multiple Sclerosis, demylenating movement disorders, spinal cord injury, asthma, chronic heart failure, obesity and stroke (particularly acute ischemia), to name a few. Typically, the systems are used to stimulate portions of neural tissue of the central nervous system, wherein the central nervous system includes the spinal cord and the pairs of nerves along the spinal cord which are known as spinal nerves. The spinal nerves include both dorsal and ventral roots which fuse in the intravertebral foramen to create a mixed nerve which is part of the peripheral nervous system. At least one dorsal root ganglion (DRG) is disposed along each dorsal root prior to the point of mixing. Thus, the neural tissue of the central nervous system is considered to include the dorsal root ganglions and exclude the portion of the nervous system beyond the dorsal root ganglions, such as the mixed nerves of the peripheral nervous system.

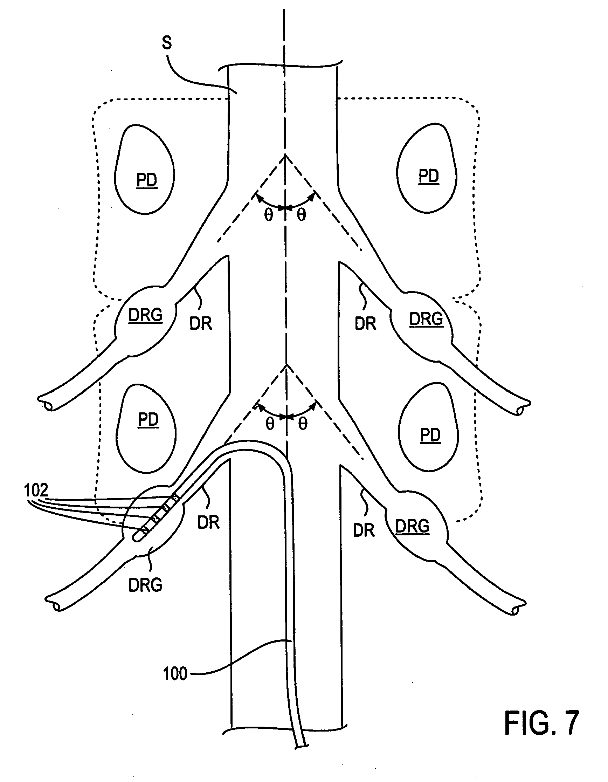

In some embodiments, the systems of the present invention are used to stimulate one or more dorsal root ganglia, dorsal roots, dorsal root entry zones, or portions thereof. Accessing these areas is challenging, particularly from an antegrade epidural approach. Fig. 7 schematically illustrates portions of the anatomy in such areas. As shown, each DRG is disposed along a dorsal root DR and typically resides at least partially between the pedicles PD or within a foramen. Each dorsal root DR exits the spinal cord S at an angle θ. This angle θ is considered the nerve root sleeve angulation and varies slightly by patient and by location along the spinal column. The average nerve root angulation in the lumbar spine is significantly less than 90 degrees and typically less than 45 degrees. Therefore, accessing this anatomy from an antegrade approach involves making a sharp turn through, along or near the nerve root sleeve angulation. It may be appreciated that such a turn may follow the nerve root sleeve angulation precisely or may follow various curves in the vicinity of the nerve root sleeve angulation.

Fig. 7 illustrates a lead 100 inserted epidurally and advanced in an antegrade direction along the spinal cord S. The lead 100, having at least one electrode 102 thereon, is advanced through the patient anatomy so that at least one of the electrodes 102 is positioned on a target DRG. Such advancement of the lead 100 toward the target DRG in this manner involves making a sharp turn along the angle θ. A turn of this severity is achieved with the use of delivery tools and design features of the present invention specific to such lead placement. In addition, the spatial relationship between the nerve roots, DRGs and surrounding structures are significantly influenced by degenerative changes, particularly in the lumbar spine. Thus, patients may have nerve root angulations which differ from the normal anatomy, such as having even smaller angulations necessitating even tighter turns. The present invention also accommodates these anatomies.

The systems of the present invention allow for targeted treatment of the desired anatomies. Such targeted treatment minimizes deleterious side effects, such as undesired motor responses or undesired stimulation of unaffected body regions. This is achieved by directly neuromodulating a target anatomy associated with the condition while minimizing or excluding undesired neuromodulation of other anatomies. For example, this may include stimulating the dorsal root ganglia, dorsal roots, dorsal root entry zones, or portions thereof while minimizing or excluding undesired stimulation of other tissues, such as surrounding or nearby tissues, portions of the ventral root and portions of the anatomy associated with body regions which are not targeted for treatment. Such stimulation is achieved with the use of a lead having at least one electrode thereon. The lead is advanced through the patient anatomy so that the at least one electrode is positioned on, near or about the target. In some embodiments, the lead and electrode(s) are sized and configured so that the electrode(s) are able to minimize or exclude undesired stimulation of other anatomies. In other embodiments, the stimulation signal or other aspects are configured so as to minimize or exclude undesired stimulation of other anatomies. In addition, it may be appreciated that stimulation of other tissues are also contemplated.

In most embodiments, neuromodulation comprises stimulation, however it may be appreciated that neuromodulation may include a variety of forms of altering or modulating nerve activity by delivering electrical stimulation directly to a target area. For illustrative purposes, descriptions herein will be provided in terms of stimulation and stimulation parameters, however, it may be appreciated that such descriptions are not so limited and may include any form of neuromodulation and neuromodulation parameters.

### System Overview

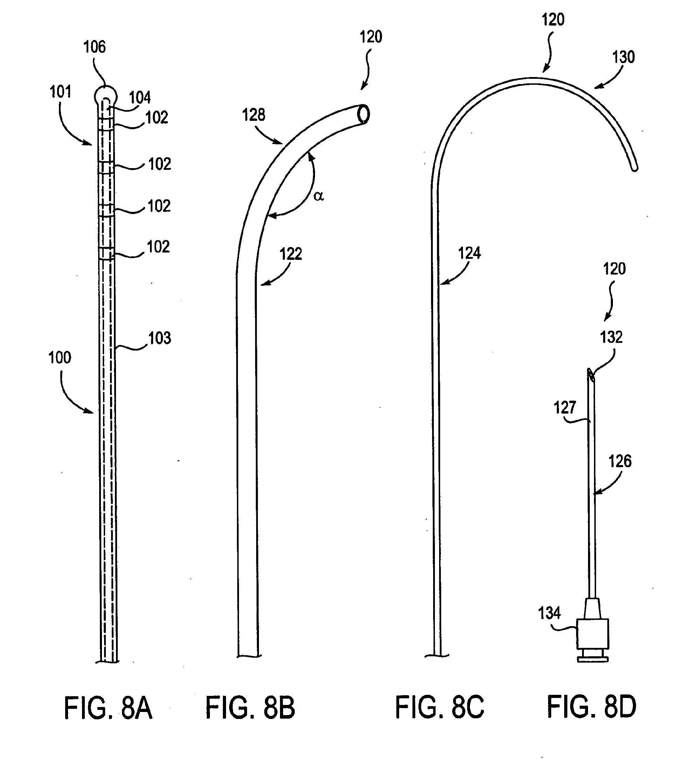

Referring to Figs. 8A-8D, an embodiment of a lead 100 (Fig. 8A) and delivery system 120, including a sheath 122 (Fig. 8B), stylet 124 (Fig. 8C) and introducing needle 126 (Fig. 8D), of the present invention is illustrated. In this embodiment, the lead 100 comprises a shaft 103 having a distal end 101 and four electrodes 102 disposed thereon. It may be appreciated that any number of electrodes 102 may be present, including one, two, three, four, five, six, seven, eight or more. In this embodiment, the distal end 101 has a closed-end distal tip 106. The distal tip 106 may have a variety of shapes including a rounded shape, such as a ball shape (shown) or tear drop shape, and a cone shape, to name a few. These shapes provide an atraumatic tip for the lead 100 as well as serving other purposes. The lead 100 also includes a stylet lumen 104 which extends toward the closed-end distal tip 106.

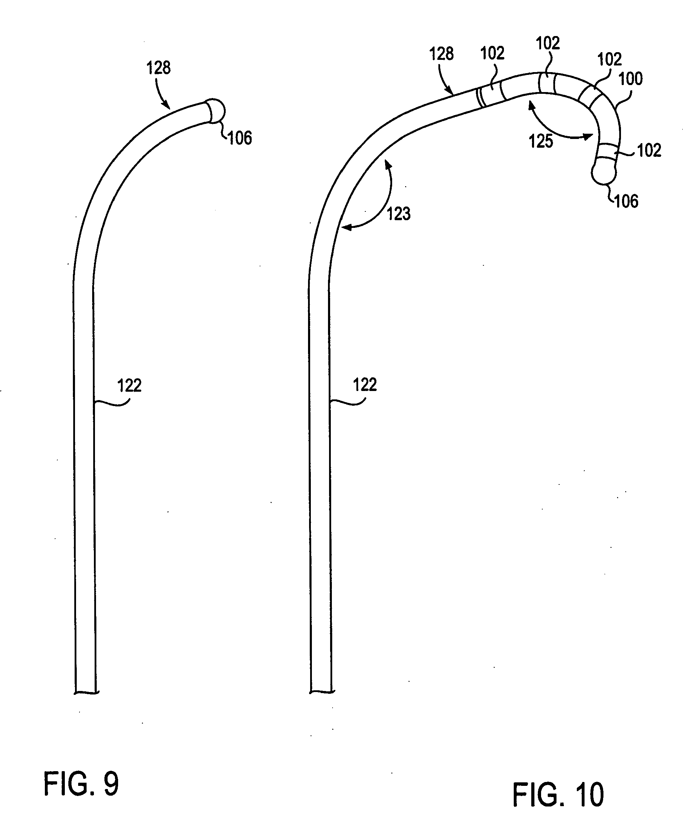

Fig. 8B illustrates an embodiment of a sheath 122 of the present system. The sheath 122 has a distal end 128 which is pre-curved to have an angle α, wherein the angle α is in the range of approximately 80 to 165 degrees. The sheath 122 is sized and configured to be advanced over the shaft 103 of the lead 100 until a portion of its distal end 128 abuts the distal tip 106 of the lead 100, as illustrated in Fig. 9. Thus, the ball shaped tip 106 of this embodiment also prevents the sheath 122 from extending thereover. Passage of the sheath 122 over the lead 100 causes the lead 100 to bend in accordance with the precurvature of the sheath 122. Thus, the sheath 122 assists in steering the lead 100 along the spinal column S and toward a target DRG, such as in a lateral direction. It may be appreciated that the angle α may optionally be smaller, such as less than 80 degrees, forming a U-shape or tighter bend.

Referring back to Fig. 8C, an embodiment of a stylet 124 of the present system is illustrated. In this embodiment, the stylet 124 has a distal end 130 which is pre-curved so that its radius of curvature is in the range of approximately 2·54 to 12·7 mm (0.1 to 0.5 inches). The stylet 124 is sized and configured to be advanced within the stylet lumen 104 of the lead 100. Typically the stylet 124 extends therethrough so that its distal end 130 aligns with the distal end 101 of the lead 100. Passage of the stylet 124 through the lead 100 causes the lead 100 to bend in accordance with the precurvature of the stylet 124. Typically, the stylet 124 has a smaller radius of curvature, or a tighter bend, than the sheath 122. Therefore, as shown in Fig. 10, when the stylet 124 is disposed within the lead 100, extension of the lead 100 and stylet 124 through the sheath 122 bends or directs the lead 100 through a first curvature 123. Further extension of the lead 100 and stylet 124 beyond the distal end 128 of the sheath 122 allows the lead 100 to bend further along a second curvature 125. When approaching a target DRG, the second curvature allows the laterally directed lead 100 to now curve around toward the target DRG, such as along the nerve root angulation. This two step curvature allows the lead 100 to be successfully positioned so that at least one of the electrodes 102 is on, near or about the target DRG, particularly by making a sharp turn along the angle θ. In addition, the electrodes 102 are spaced to assist in making such a sharp turn.

Thus, the lead 100 does not require stiff or torqueable construction since the lead 100 is typically not torqued or steered by itself. The lead 100 is positioned with the use of the sheath 122 and stylet 124 which direct the lead 100 through the two step curvature. This eliminates the need for the operator to torque the lead 100 and optionally the sheath 122 with multiple hands. This also allows the lead 100 to have a lower profile and smaller diameter, as well as a very soft and flexible construction. This, in turn, minimizes erosion, irritation of the neural tissue and discomfort created by pressure on nerve tissue, such as the target DRG and/or the nerve root, once the lead 100 is implanted. In addition, such a soft and flexible lead 100 will minimize the amount of force translated to the distal end of the lead 100 by body movement (e.g. flexion, extension, torsion).

Referring back to Fig. 8D, an embodiment of an introducing needle 126 is illustrated. The introducing needle 126 is used to access the epidural space of the spinal cord S. The needle 126 has a hollow shaft 127 and typically has a very slightly curved distal end 132. The shaft 127 is sized to allow passage of the lead 100, sheath 122 and stylet 124 therethrough. In some embodiments, the needle 126 is 14 gauge which is typically the size of epidural needles used to place conventional percutaneous leads within the epidural space. However, it may be appreciated that other sized needles may also be used, particularly smaller needles such as 15-18 gauge. Alternatively, non-standardized sized needles may be used.

The needle is atraumatic so as to not damage the sheath 122 when the sheath 122 is advanced or retracted. In some embodiments, the shaft 127 comprises a low friction material, such as bright hypotubing, made from bright steel (a product formed from the process of drawing hot rolled steel through a die to impart close dimensional tolerances, a bright, scale free surface and improved mechanical properties. Other materials include polytetrafluoroethylene (PTFE) impregnated or coated hypotubing. In addition, it may be appreciated that needles having various tips known to practitioners or custom tips designed for specific applications may also be used. The needle 126 also typically includes a luer fitting 134, such as a Luer-Lok™ fitting, or other fitting near its proximal end. The luer fitting 134 is a female fitting having a tabbed hub which engages threads in a sleeve on a male fitting, such as a syringe. The needle 126 may also have a luer fitting on a side port, so as to allow injection through the needle 126 while the sheath 122 is in the needle 126. In some embodiments, the luer fitting is tapered to allow for easier introduction of a curved sheath into the hollow shaft 127.

### Delivery Methods

The above described delivery system 120 is used for epidural delivery of the lead 100 through the patient anatomy toward a target DRG. Thus, embodiments of epidural delivery methods are described herein. In particular, such embodiments are described and illustrated as an antegrade approach. It may be appreciated that, alternatively, the devices and systems of the present invention may be used with a retrograde approach or a contralateral approach. Likewise, at least some of the devices and systems may be used with a transforaminal approach, wherein the DRG is approached from outside of the spinal column. Further, the target DRG may be approached through the sacral hiatus or through a bony structure such as a pedicle, lamina or other structure.

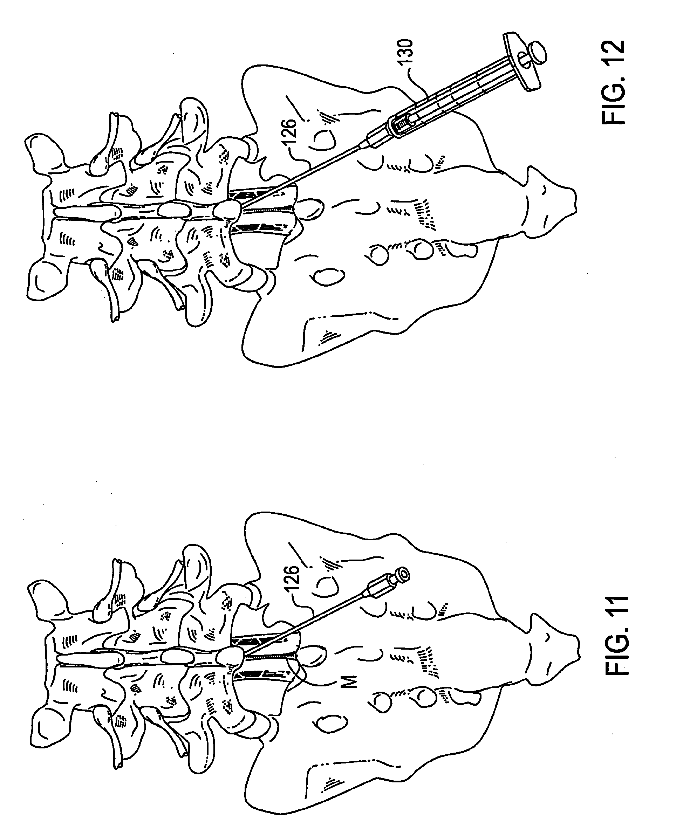

Epidural delivery involves accessing the epidural space. The epidural space is accessed with the use of the introducing needle 126, as illustrated in Fig. 11. Typically, the skin is infiltrated with local anesthetic such as lidocaine over the identified portion of the epidural space. The insertion point is usually near the midline M, although other approaches may be employed. Typically, the needle 126 is inserted to the ligamentum flavum and a loss of resistance to injection technique is used to identify the epidural space. Referring to Fig. 12, a syringe 140 is then attached to the needle 126. The syringe 140 may contain air or saline. Traditionally either air or saline has been used for identifying the epidural space, depending on personal preference. When the tip of the needle 126 enters a space of negative or neutral pressure (such as the epidural space), there will be a "loss of resistance" and it will be possible to inject through the syringe 140. At that point, there is now a high likelihood that the tip of the needle 126 has entered the epidural space. Further, a sensation of "pop" or "click" may be felt as the needle breaches the ligamentum flavum just before entering the epidural space. In addition to the loss of resistance technique, realtime observation of the advancing needle 126 may be achieved with a portable ultrasound scanner or with fluoroscopy. Likewise, a guidewire may be advanced through the needle 126 and observed within the epidural space with the use of fluoroscopy.

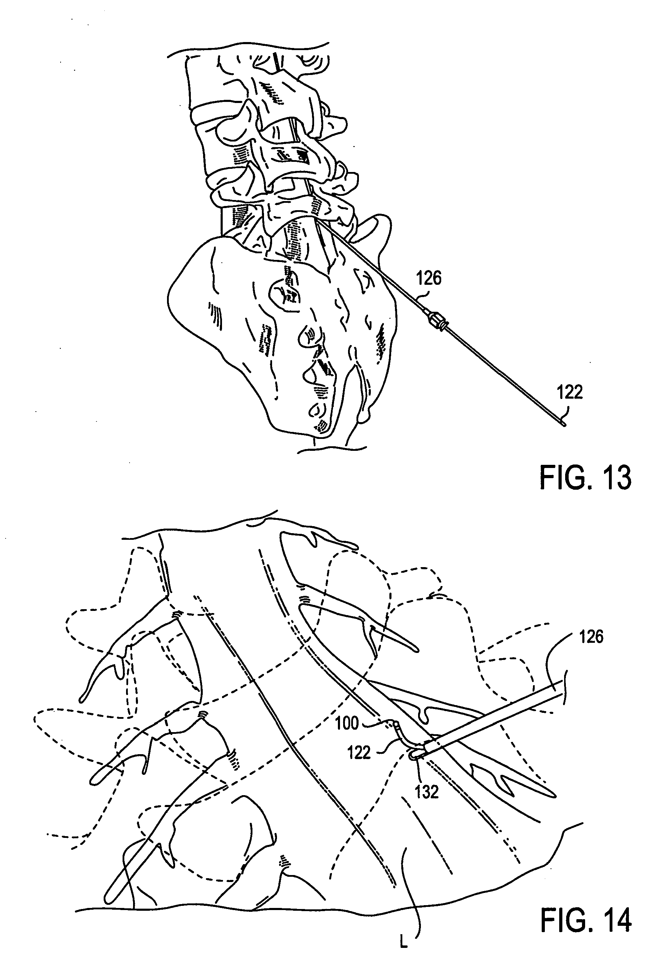

Once the needle 126 has been successfully inserted into the epidural space, the syringe 140 is removed. The stylet 124 is inserted into the lead 100 and the sheath 122 is advanced over the lead 100. The sheath 122 is positioned so that its distal end 128 is near or against the distal tip106 of the lead 100 causing the lead 100 to follow the curvature of the sheath 122. The stylet 124, lead 100 and sheath 122 are then inserted through the needle 126, into the epidural space, as illustrated in Fig. 13. Referring to Fig. 14, the distal end 132 of the needle 126 is shown passed through the ligamentum flavum L and the assembled sheath 122/lead 100/stylet 124 is shown emerging therefrom. The rigidity of the needle 126 straightens the more flexible sheath 122 as it passes therethrough. However, upon emergence, the sheath 122 is allowed to bend along or toward its precurvature as shown. In some embodiments, the shape memory of the sheath 122 material allows the sheath 122 to retain more than 50% of its precurved shape upon passing through the needle 126. Such bending assists in steering of the lead 100 within the epidural space. This is particularly useful when using a retrograde approach to navigate across the transition from the lumbar spine to the sacral spine. The sacrum creates a "shelf" that resists ease of passage into the sacrum. The precurved sheath 122 is able to more easily pass into the sacrum, reducing operating time and patient discomfort.

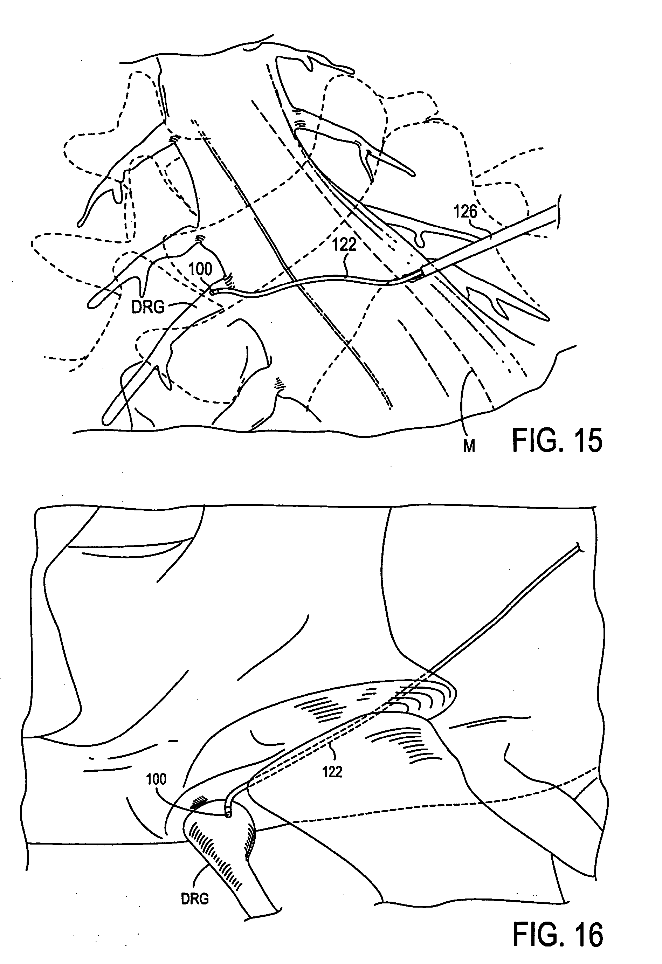

Referring to Fig. 15, the assembled sheath 122/lead 100/stylet 124 is advanced within the epidural space toward a target DRG. Steering and manipulation is controlled proximally and is assisted by the construction of the assembled components and the precurvature of the sheath 122. In particular, the precurvature of the sheath 122 directs the lead 100 laterally outwardly, away from the midline M of the spinal column. Fig. 16 illustrates the assembled sheath 122/lead 100/stylet 124 advanced toward the target DRG with the precurvature of the sheath 122 directing the lead 100 laterally outwardly.

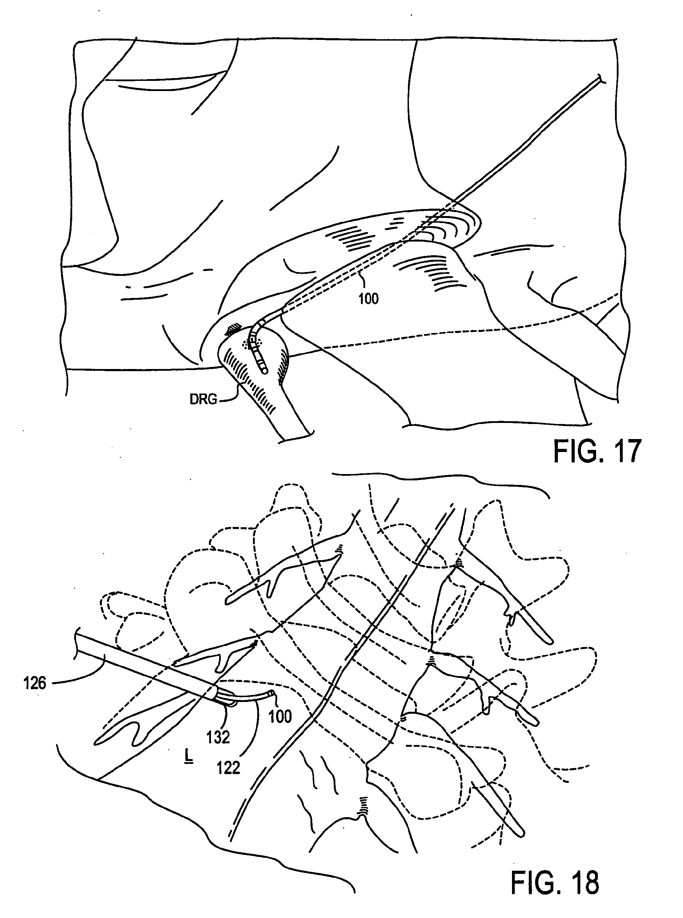

Referring to Fig. 17, the lead 100/stylet 124 is then advanced beyond the distal end 128 of the sheath 122. In some embodiments, the lead 100 extends approximately 2.54-7.62 cm (1-3 inches) beyond the distal end 128 of the sheath 122. However, the lead 100 may extend any distance, such as less than 2.54 cm (1 inch), 0.64-7.62 cm (0.25-3 inches), or more than 7.62 cm (3 inches). Likewise, the sheath 122 may be retracted to expose the lead 100, with or without advancement of the lead 100. This may be useful when advancement of the lead 100 is restricted, such as by compression of the foraminal opening. The curvature of the stylet 124 within the lead 100 causes the lead 100 to bend further, along this curvature. This allows the laterally directed lead 100 to now curve around toward the target DRG along the nerve root angulation. This two step curvature allows the lead 100 to be successfully steered to position at least one of the electrodes 102 on, near or about the target DRG. In addition, the ball shaped distal tip 106 resists trauma to the anatomy within the spinal column, such as the dural sac, ligaments, blood vessels, and resists imparting trauma to the DRG as the lead 100 is manipulated and advanced into place. Once desirably positioned, the sheath 122 and stylet 124 are typically removed leaving the lead 100 in place. However, optionally, the stylet 124 may be left within the lead 100 to stabilize the lead 100, to assist in maintaining position and to resist migration. The DRG may then be stimulated by providing stimulation energy to the at least one electrode 102, as illustrated by energy ring 140 in Fig. 17. It may be appreciated that multiple electrodes may be energized to stimulate the target DRG. It may also be appreciated that the electrodes may be energized prior to removal of the stylet 124 and/or sheath 122, particularly to ascertain the desired positioning of the lead 100. It may further be appreciated that the sheath 122 may be retracted to expose the lead 100 rather than advancing the lead 100 therethrough.

The same needle 126 can then be used to position additional leads within the epidural space. Again, a stylet 124 is inserted into a lead 100 and a sheath 122 is advanced over the lead 100. The sheath 122 is positioned so that its distal end 128 is near or against the distal tip106 of the lead 100 causing the lead 100 to follow the curvature of the sheath 122. The assembled stylet 124/lead 100/sheath 122 is then inserted through the needle 126, into the epidural space, as illustrated in Fig. 18. The rigidity of the needle 126 straightens the more flexible sheath 122 as it passes therethrough. And, upon emergence, the sheath 122 is allowed to bend along its precurvature as shown. This creates an atraumatic exit of the stylet 124/lead 100/sheath 122 out of the needle 126 since such curvatures resist any directed force into the dura layer of the spinal cord. This also assists in steering of the lead 100 within the epidural space.

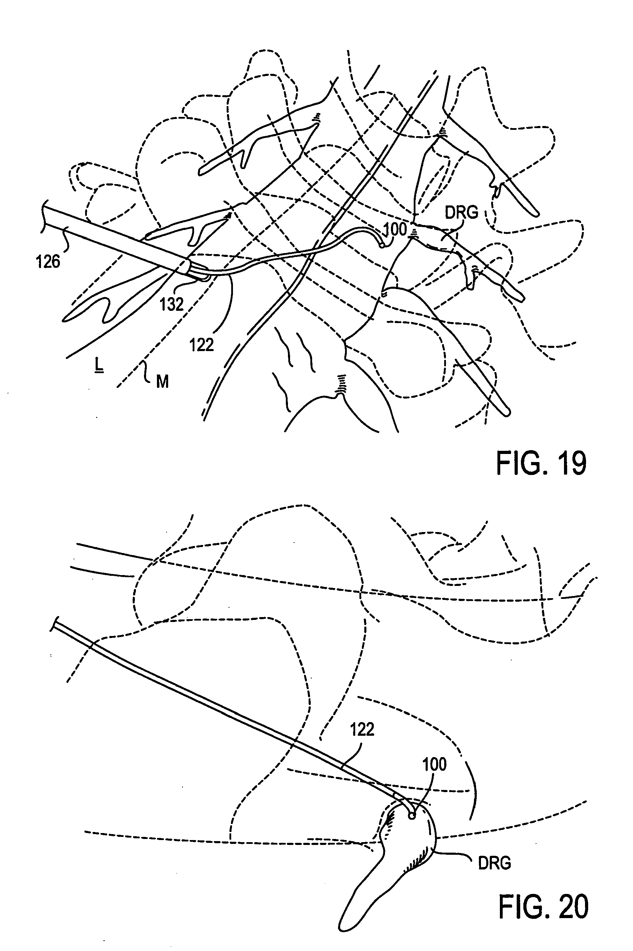

Referring to Fig. 19, the assembled sheath 122/lead 100/stylet 124 is advanced within the epidural space toward another or second target DRG. In this embodiment, the second target DRG is on an opposite side of the spinal column from the first target DRG. Again, the precurvature of the sheath 122 can be used to steer the lead 100 and direct the lead 100 laterally outwardly, away from the midline M of the spinal column. Thus, DRGs on each side of the spinal column can be accessed by manipulation of the sheath 122 while entering the epidural space from the same insertion point. Fig. 20 illustrates the assembled sheath 122/lead 100/stylet 124 advanced toward the second target DRG with the precurvature of the sheath 122 directing the lead 100 laterally outwardly.

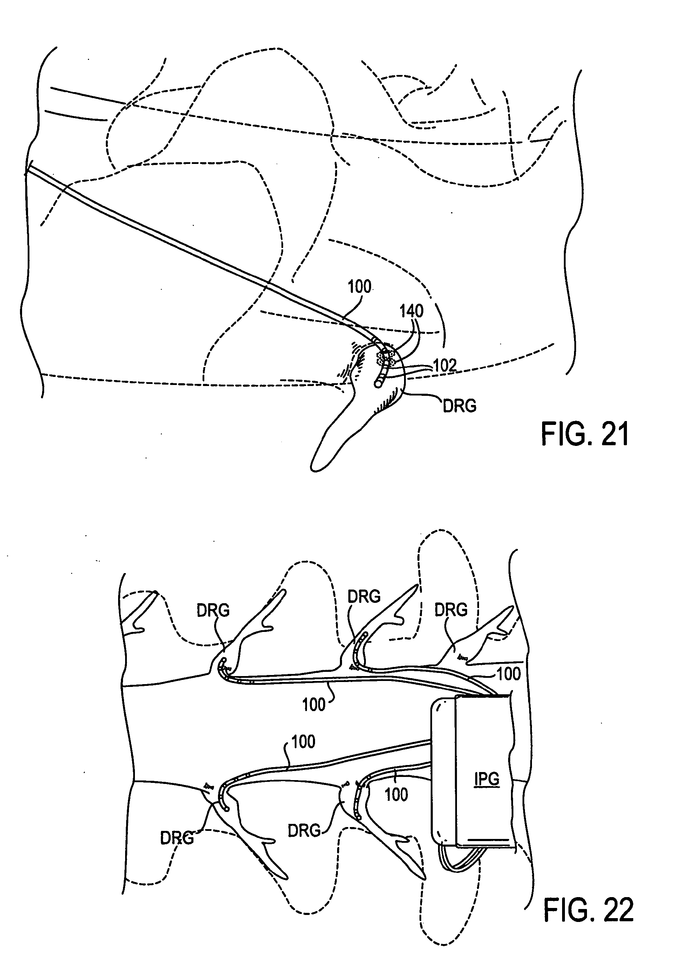

The lead 100/stylet 124 is then advanced beyond the distal end 128 of the sheath 122. Again, the curvature of the stylet 124 within the lead 100 causes the lead 100 to bend further, along this curvature. This allows the laterally directed lead 100 to now curve around toward the target DRG along the nerve root angulation. This two step curvature allows the lead 100 to be successfully steered to position at least one of the electrodes 102 on, near or about the target DRG. Once desirably positioned, the sheath 122 and stylet 124 are removed leaving the lead 100 in place, as illustrated in Fig. 21. The DRG may then be stimulated by providing stimulation energy to the at least one electrode 102, as illustrated by energy rings 140 in Fig. 21. Again, it may be appreciated that multiple electrodes may be energized to stimulate the target DRG. It may also be appreciated that the electrodes may be energized prior to removal of the stylet 124 and/or sheath 122, particularly to ascertain the desired positioning of the lead 100.

It may be appreciated that any number of leads 100 may be introduced through the same introducing needle 126. In some embodiments, the introducing needle 126 has more than one lumen, such as a double-barreled needle, to allow introduction of leads 100 through separate lumens. Further, any number of introducing needles 126 may be positioned along the spinal column for desired access to the epidural space. In some embodiments, a second needle is placed adjacent to a first needle. The second needle is used to deliver a second lead to a spinal level adjacent to the spinal level corresponding to the first needle. In some instances, there is a tract in the epidural space and the placement of a first lead may indicate that a second lead may be easily placed through the same tract. Thus, the second needle is placed so that the same epidural tract may be accessed. In other embodiments, a second needle is used to assist in stabilizing the tip of a sheath inserted through a first needle. In such embodiments, the second needle is positioned along the spinal column near the target anatomy. As the sheath is advanced, it may use the second needle to buttress against for stability or to assist in directing the sheath. This may be particularly useful when accessing a stenosed foramen which resists access.

Fig. 22 illustrates a plurality of leads 100 positioned within the epidural space, each lead 100 stimulating a different DRG. In this example, the DRGs are on multiple levels and on both sides of the spinal column. The proximal ends of the leads 100 are connected with an IPG (shown in part) which is typically implanted nearby.

Thus, delivery of the lead 100 of the present system through the patient anatomy toward a target DRG involves more potential challenges than delivery of conventional spinal cord stimulator leads. For example, one significant challenge is steering the lead 100 within the epidural space, particularly laterally toward the target DRG and curving the lead 100 through the nerve root sleeve angulation to position at least one of the electrodes 106 on, near or about the DRG. In addition, such leads 100 should be atraumatic and resist kinking, migration fracture or pullout while implanted. Therefore, significant floppiness and flexibility is desired. However, a more flexible lead can be more difficult to manipulate. To overcome these conflicting challenges, a variety of design features have been incorporated into the devices.

### Lead and Delivery Devices

As described above, the present invention includes a variety of devices, including one or more leads 100 and a delivery system 120, including a sheath 122, stylet 124 and introducing needle 126.

In some embodiments, the introducing needle 126 is a standard epidural access device used commonly with an anti-coring stylet. Such needles 126 are typically comprised of stainless steel and have an atraumatic tip to prevent insertion through the spinal dural sac. In some embodiments, the introducing needle is a 14 gauge thin-wall, however it may be appreciated that other sized needles may be used, particularly smaller diameter needles.

The sheath 122, lead 100 and stylet 124 are all passable through the needle 126 for introduction to the epidural space without damage to the needle 126 or to the devices passed therethrough. Thus, access can be achieved through a single entry point and the devices can be advanced, retracted, removed and reinserted through the needle 126 with ease and without irritation, injury or disruption to the tissues surrounding the entry point. This provides a significant improvement over conventional delivery systems which recommend introduction of devices using a Seldinger Technique. When using the Seldinger Technique, a guidewire is passed through the introducing needle and the needle is withdrawn. A conventional delivery sheath is then advanced over the guidewire into the epidural space. The guidewire is then removed and the sheath is used as a conduit for delivery of devices to the epidural space. However, the tip of the sheath tends to fold and irritate the patient during placement through the ligamentum flavum. Also, the conventional sheath lacks the column strength to push through calcified or difficult to pass tissue. Further, the introduction and removal of each of these devices increases the risk of dural puncture and patient discomfort. Consequently, conventional sheaths are typically abandoned in favor of directly advancing the lead into the epidural space. This may be possible since conventional lead placement simply involves linear advancement along the spinal column without significant steering, bending or curving and conventional sheaths provide no guiding or steering capability anyway. Conventional sheaths are also incapable of fitting through a conventional introducing needle due to their size and wall thickness. Thus, practitioners are left with manipulating the lead itself.

### Sheath

The sheath 122 of the present system comprises a hollow tube having a stiffness which allows advancement along the epidural space. In some embodiments, such stiffness has a minimum of approximately 4.48 kPa (0.65 lbs·in²) and a maximum of approximately 15.5 kPa (2.25 lbs·in²). Thus, in some embodiments, the sheath 122 has a stiffness of approximately 12.48 kPa (1.81 lbs·in²).

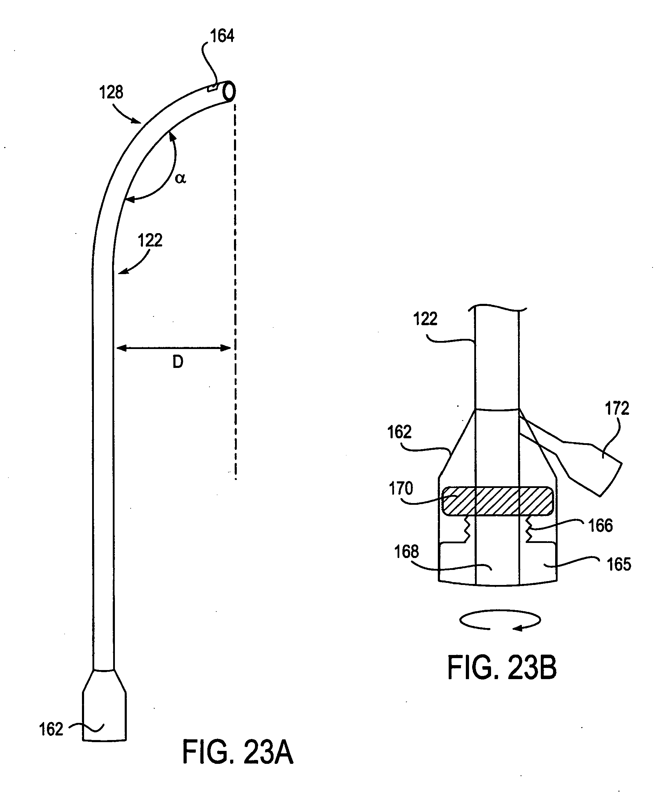

In most embodiments, the sheath 122 has a preformed or preset bend near its distal end 128, as illustrated in Fig. 23A, to assist in accessing the target anatomy. In some embodiments, the bend has an angle α of approximately 15-165 degrees, however any suitable angle may be used. The bend can also be characterized by the lateral distance D from the distal tip to the outer surface of the shaft, as illustrated in Fig. 23A. In some embodiments, the distance D is approximately 0.76-9.53 mm (0.030-0.375 inches). In some embodiments, the sheath 122 is sized and shaped for particular types of delivery, such as antegrade, retrograde, and contralateral approaches, to name a few. In some embodiments, an antegrade sheath (configured for antegrade delivery) has a bend with an angle α of approximately 90-110 degrees and a distance D of approximately 8.25-9.53 mm (0.325-0.375 inches). Bends having an angle α less than or equal to150 degrees and a distance D of greater than or equal to 5.72 mm (0.225 inches) typically improve the ease of delivery when using an antegrade approach to the DRG. In some embodiments, an alternate sheath (configured for retrograde or contralateral delivery) has a bend with an angle α of approximately 130-150 degrees and a distance D of approximately 1.14-2.4 mm (0.045-0.095 inches). Bends having an angle α less than or equal to 165 degrees and a distance D of greater than or equal to 0.76 mm (0.030 inches) typically improve the ease of delivery when using a retrograde or contralateral approach to the DRG. The sheath 122 can be rigid enough to guide the lead 100/stylet 124 without the sheath 122 significantly deflecting. Alternatively, the sheath 122 may be more flexible to allow increased steering or guiding through the anatomy.

Typically, the sheath 122 is comprised of a polymer, such as polyimide, or polyetheretherketone (PEEK). In preferred embodiments, the sheath 122 is comprised of a plastic material, such as a thermoset and/or thermoplastic material. Polyimide is preferred due to the thinness of its walls while retaining high strength, superior shape memory and shape retention. Polyimide can also be straightened for passage through the introducing needle 126 without kinking. In some embodiments, the sheath 122 is comprised of polyimide material having a wall thickness in the range of approximately 0·051-0·152 mm (0·002-0·006 inches), more particularly approximately 0·076-0·152 mm (0·003-0·006 inches). It may be appreciated that other materials may be used provided the resulting sheath has an appropriate stiffness to allow advancement along the epidural space, while having a wall-thickness thin enough to allow passage of the sheath and lead through an introducing needle to the epidural space, and while having a sufficiently low coefficient of friction to allow desirable passage of the lead therethrough. Further, the resulting sheath should be kink-resistant and formable into a desired shape. Examples of other materials potentially meeting these criteria include nylon, polycarbonate, acrylonitrile butadiene styrene (ABS), Polyethylene terephthalate (PET) and Pebax, to name a few.

Typically, the sheath 122 is comprised of a single stiffness or unidurometer material. This is possible because the sheath 122, lead 100 and stylet 124 are introduced together to the epidural space, sharing the delivery workload. In particular, since the lead 100 and stylet 124 substantially fill the inner diameter of the sheath 122, strength and kink resistance are bolstered for delivery robustness. In contrast, if the sheath 122 were introduced alone, stiffness transitions, such as durometer/materials changes, or reinforcements, such as braiding, may be needed for kink resistance. However, it may be appreciated that sheath 122 may optionally be comprised of a reinforced polymer, such as a braided polymer, or may be comprised of a construct of various materials. For example, the tip of the sheath 122 may be comprised of a differing material or a thinner material to create a less traumatic or an atraumatic tip. Such a tip may be more flexible than the remainder of the sheath which provides increased torqueability and pushability. Further it may be appreciated that the sheath 122 may optionally be comprised of a flexible metal or metal/polymer construct.

Delivery of the lead 100, stylet 124 and sheath 122 together also provides a number of other benefits. For example, preloading of the lead 100, stylet 124 and sheath 122 and simultaneous delivery eliminates multiple steps and complications associated with separate introduction of each device. Further, matching the coaxial shapes of the lead 100, stylet 124 and sheath 122 create steerability and lead control without the need for stiffening lead construction and without sacrificing lead flexibility and profile. In addition, preloading of the sheath 122 with a lead 100 having a ball shaped distal tip 106 allows the sheath 122 to have a comparatively hard or sharp tip because it is shielded by the atraumatic ball shape of the distal tip 106 of the lead 100. Thus, the practitioner may be less concerned with traumatizing surrounding tissue during delivery in comparison to advancing a traditional open-tipped sheath. However, it may be appreciated that the distal end of the sheath 122 may optionally be formed from a soft material, such as Pebax, to create a more atraumatic tip for the sheath 122 itself. In such instances, the sheath 122 may optionally be used with a lead 100 without a ball shaped distal tip 106 and may be loaded on the lead 100 either from the proximal or distal ends of the lead 100.

The ball shaped distal tip 106 of the lead 100 also provides tactile feedback when retracted against the sheath 122. Such feedback allows the practitioner to tactilely determine the relative position of the lead 100 to the sheath 122. It may be appreciated that other mechanisms may be used to register the distal tip 106 of the lead 100 against the sheath 122, such as slots, pins, and bands, to name a few. Alternatively, such registering may be achieved near the proximal end of the lead 100 and sheath 122.

In some embodiments, the sheath 122 includes a chamfer or flared edge near its distal end to assist in retraction of the lead 100 therein. In some instances, the chamfer comprises radiusing of the inside of the sheath 122 near the distal end by, for example, approximately 0·051 mm (0·002 inches) or more. Such radiusing provides an atraumatic, smooth edge to funnel the lead 100 and electrodes 102 thereon into the sheath 122. Likewise, a flared edge assists in allowing the lead 100 and electrodes 102 thereon to pass into the sheath 122 without hooking on the distal end of the sheath 122. This reduces any risk of damage to the lead 100, such as due to the electrodes 102 catching on the sheath 122, and reduces procedure time since the physician can reposition the device without removing the entire system.

In most embodiments, the sheath 122 also includes a hub 162, such as illustrated in Fig. 23A, near its proximal end wherein the hub 162 assists in manipulation of the sheath 122. The torsional rigidity of the sheath 122 allows the sheath 122 to be torqued by rotation of the hub 162. In some embodiments, the hub 162 also provides indication of the direction of the bend. This assists in steering the lead 100 with or without the aid of visualization. In instances where visualization is used, such as fluoroscopy, an embodiment of the sheath 122 may be used which has a radiopaque marker 164 near its distal end 128. Alternatively, the sheath 122 may be marked with radiopaque stripes, such as along the distal end 128 or along the length of the sheath 122. Likewise, the sheath 122 may be marked with radiopaque marker bands, such as tungsten or platinum marker bands, since the wall thickness of the sheath 122 is not limited by the epidural space.

Alternatively or in addition, the sheath 122 may be loaded with radiopaque material to provide radiopacity along the distal end 128 or along its length. In any case, any suitable radiopaque material may be used, such as tungsten or barium sulfate. In some embodiments, the sheath 122 is less radiopaque than the lead 100 so that the practitioner can maintain visualization of the lead 100 and can visualize the interaction of the sheath 122 and lead 100 together. Or, in some embodiments, the sheath 122 and lead 100 each have radiopaque markers at their respective ends so that the practitioner is aware of their locations, both within the anatomy and in relation to each other. Visualization of the lead 100 and sheath 122 is particularly useful for the methods disclosed herein which typically involve manipulation of the devices in three dimensions, such as movement in and out of different planes, as opposed to conventional SCS lead placement which occurs in two dimensions.

Such movement of the lead 100, including curving of the lead 100 through the nerve root sleeve angulation, typically involves more and greater bends (bends having lower radii) to the distal end 101 of the lead 100 than conventional leads used in standard SCS therapy. Consequently, embodiments of the lead 100 of the present system have a variety of design features to accommodate such bending and increased manipulation demands. Typically, the lead 100 has a more flexible distal end 101 than conventional leads and has a lower diameter. Most embodiments of the lead 100 also minimize constraints on internal components and utilize low stiffness materials. Such features ease manipulation, reduce any possibility of trauma to the DRG and resist lead migration since less load and strain from the body will be translated to the distal end of the lead itself.

Referring to Fig. 23B, in some embodiments the hub 162 includes a locking cap 165 which is used to lock the lead 100 in position within the sheath 122. Such locking may assist in reducing movement of the lead 100 during manipulation of the sheath 122. In one embodiment, the locking cap 165 has a threaded elongated portion 166 which engages with threads within the hub 162. The locking cap 165 also has an aperture 168 which aligns with a lumen extending through the sheath 122. The lead 100 is advanceable through the aperture 168 and into the lumen of the sheath 122. When the lead 100 is desirably positioned, the lead 100 may be locked in place by rotating the locking cap 165 which advances the threaded elongated portion 166 into the hub 162 and compresses a gasket 170. The gasket 170 may be comprised of any flexible material, such a silicone. Compression of the gasket 170 causes the gasket 170 to engage the lead 100, thereby locking the lead 100 in place by frictional forces. Optionally, the hub 162 may include an injection port 172 which may be used to inject a desired medium, such as contrast, saline or other fluids.

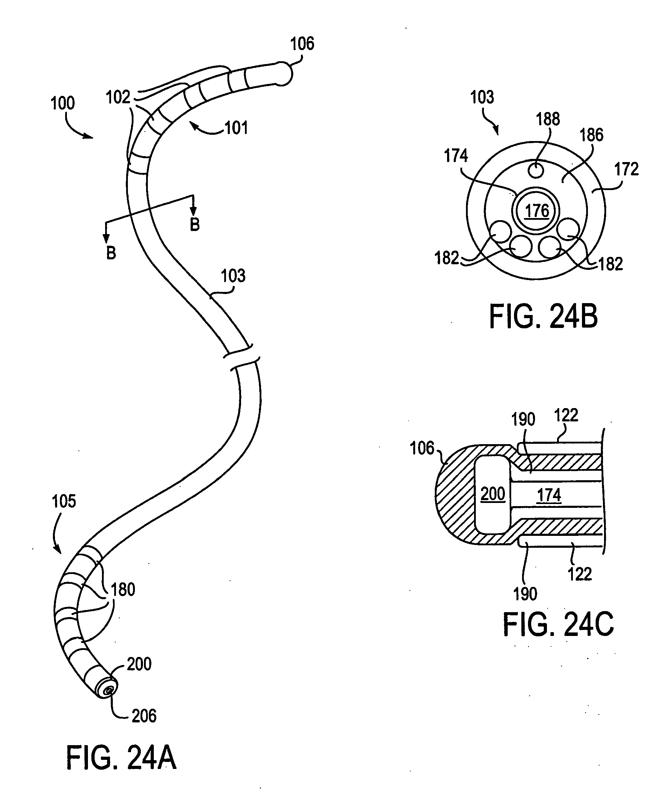

Figs. 24A-24E illustrate an embodiment of a lead 100 of the present system. Fig. 24A provides a perspective view of an embodiment of a lead 100. The lead 100 comprises a shaft 103 having a distal end 101 and a proximal end 105. In this embodiment, the shaft 103 comprises a single lumen tube 172 formed from an extruded polymer, such as urethane. Fig. 24B provides a cross-sectional view of the shaft 103 of Fig. 24A. Typically, the tube 172 has an outer diameter in the range of approximately 1.02-1.27 mm (0.040-0.050 inches), a wall thickness in the range of approximately 0.127-0.254 mm (0.005-0.010 inches) and a length of approximately 30.48-76.2 cm (12-30 inches), however such dimensions serve only as an example. For instance, in other embodiments, the tube 172 has an outer diameter in the range of approximately 0.71-1.27 mm (0.028-0.050 inches), a wall thickness in the range of approximately 0.076-0.254 mm (0.003-0.010 inches) and a length of approximately 30-120cm. It may be appreciated that other materials may be used, such as silicone or other commonly used implantable polymers.

Referring to Fig. 24B, the lead 100 also includes a stylet tube 174 disposed within the single lumen tube 172. The stylet tube 174 forms a stylet lumen 176 and isolates the stylet 124 from the other components of the lead 100. The stylet tube 174 also provides a smooth or lubricious surface against which the stylet 124 passes during insertion and retraction. Such lubriciousness is desirable to resist jamming or hang-ups of the highly curved stylet 124 within the lead 101. In addition, the lubricious surface reduces the effects on delivery of contamination by bodily fluids. The stylet tube 174 may also provide tensile strength to the lead 100 during delivery.

In some embodiments, the stylet tube 174 is comprised of polyimide. Polyimide is a biocompatible, high strength, smooth, flexible material. Smoothness is provided by the means of manufacturing, and adequate lubriciousness is provided by the low coefficient of friction (0.7) of the material. In some embodiments the polyimide is combined with Teflon to lower the coefficient of friction while maintaining high strength. Because polyimide is high strength, tough and smooth, stylets 124 having highly radiused bends are easier to introduce and manipulate therein without the stylet 124 catching, hanging, jamming or piercing into or through the sides of the stylet tube 174 as may occur with some polymers. In some embodiments, the polyimide material is loaded with a strengthening material to increase its overall tensile strength. Examples of such strengthening materials include engineering fibers, such as Spectra® fiber, Vectran™ fiber and Kevlar® fiber, to name a few.

The physical qualities of the polyimide material also allows the stylet lumen walls to be very thin, such as approximately 0·0254 mm (0·001 inches) or less, which helps to minimizes the overall diameter of the lead 100. Such thinness may not be achieved with the use of some other biocompatible polymer materials with equivalent strength and resistance to buckling.

In other embodiments, the stylet tube 174 is comprised of polyetheretherketone (PEEK). PEEK is a biocompatible, high strength, and smooth material, and in a thin-walled tube configuration is a sufficiently flexible material. Smoothness is provided by the means of manufacturing, and adequate lubriciousness is provided by the fairly low coefficient of friction (0.35) of the material. Because PEEK is high strength, tough and smooth, stylets 124 having highly radiused bends are easier to introduce and manipulate therein without the stylet 124 catching, hanging, jamming or piercing into or through the sides of the stylet tube 174 as may occur with some polymers.

And, in other embodiments, the stylet tube 174 is comprised of other polymers, such as Polyethylene Terephthalate (PET) film (also known as polyester or Mylar), or other materials, such as a metal tube, a flexible metal tube (such as formed from nitinol), a laser-cut metal tube, a spring or coil (such as a metal close-coiled spring), or a combination of materials and forms.

As mentioned above, the stylet tube 174 may have a lubricious surface, such as a coating or embedded layer, along at least a portion of the stylet lumen 176 to provide the desired lubriciousness. An example of such a surface is a polytetrafluoroethylene (PTFE) or parylene coating. The tube 174 may be comprised of a material such as polyimide and additionally coated, or the tube 174 may be comprised of a less lubricious material and coated to attain the desired lubricity. Such a coating may be particularly useful when the shaft 103 is comprised of a multi-lumen extrusion.

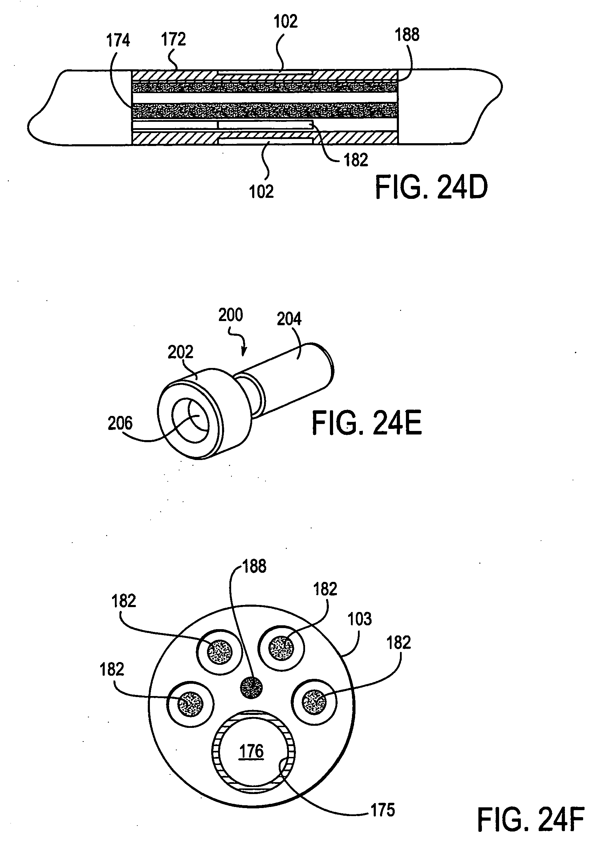

It may be appreciated that alternatively, a multi-lumen tube may be used for the shaft 103 of the lead 100, or a combination of multi-lumen and single lumen tubing. When such a multi-lumen tube is formed from an extruded polymer, various other components of the lead 100 may be coextruded with the multi-lumen tube (such as conductor cables, a stylet tube and/or a tensile wire described herein below). Fig. 24F illustrates an embodiment of a shaft 103 of the lead 100, wherein the shaft 103 comprises a 5 lumen extrusion. Four of the lumens house conductor cables 182; each conductor cable 182 loosely filling each lumen. And, one larger lumen serves as the stylet lumen 176. Typically, the stylet lumen 176 includes a lubricious surface 175, such as a coating or embedded layer, along at least a portion of the stylet lumen 176 to provide the desired lubriciousness. In addition a tensile element 188 may be co-extruded with the extrusion, as shown, or the tensile element may be loosely embedded in a sixth lumen of the extrusion. The ability to per-insert a cable or element loosely into a small lumen is a specialized aspect that allows the lead 100 increased flexibility. And, although the lead 100 is typically curved by devices such as a stylet, the distal end of the multi-lumen tube may optionally be thermally precurved to assist in such curvatures.

Referring back to Fig. 24A, the lead 100 also includes at least one electrode 102. In this embodiment, the lead 100 includes four electrodes 102 disposed along its distal end 101. Typically, the electrodes 102 are comprised of platinum or platinum/iridium alloy. In this embodiment, the electrodes 102 have a ring shape, extending around the shaft 103, and have an outer diameter approximately equal to the outer diameter of the shaft 103. In some embodiments, the electrodes have a wall thickness of approximately 0·051-0·102 mm (0·002-0·004 inches) and a length of approximately 0·16-1·52 mm (0·030-0·060 inches) or greater. It may be appreciated that the shaped distal tip 106 of the lead 100 may be formed from the most distal electrode. And, it may be appreciated a proximal end cap (described below) may serve as the most proximal electrode.

The lead 100 also includes at least one electrical contact 180 disposed near its proximal end 105 which is removably connectable with a power source, such as an implantable pulse generator. In this embodiment, the lead 100 includes a corresponding electrical contact 180 for each electrode 102. Electrical energy is transmitted from the electrical contact 180 to the corresponding electrode 102 by a conductor cable 182 which extends therebetween. Thus, the cables 182 are typically approximately 45·7-55·9 cm (18-22 inches) long, but are typically up to 120 cm (47.24 inches) long.

Referring to Fig. 24B, the conductor cables 182 extend through a space 186 between the stylet tube 174 and the single lumen tube 172. The cables 182 may be comprised of any suitable material, preferably multiple Drawn Filled Tube (DFT) strands each comprising a high strength outer layer of cobalt-chrome alloy and a high conductivity core of silver, platinum or platinum/iridium alloy. Typically, the cables 182 are electrically insulated by a thin layer of material, such as polytetrafluoroethylene (PTFE) or perfluoroalkoxy (PFA). Consequently, the cables 182 typically have an outer diameter of approximately 0·15 mm (0.006 inches). However, it may be appreciated that the cables 182 may be uncoated or uninsulated when the shaft 103 is comprised of a multi-lumen extruded tube and each cable 182 extends through a dedicated lumen, or alternatively, when the cables are embedded in the wall of the extruded tube. Another type of cable construction can include a combination of high strength strands and high conductivity strands. Alternatively, only high strength strands, such as cobalt-chrome alloy or stainless steel, may be used. In such embodiments, resistance may be decreased by enlarging the cable cross section.

Each cable 182 is joined to an electrode 102 and a corresponding electrical contact 180 by a suitable method, such as welding, brazing, soldering or crimping, to name a few. The joining process provides an electrical contact between the cable and the electrode, and also resists separation of the cable from the electrode due to any tensile forces that the lead may be subjected to during or after implantation. Therefore, the joining process should be electrically low resistance and be physically high strength. A high strength joint is enabled by ensuring that neither of the materials being joined are degraded by the joining process, in addition to having sufficient surface area, compatible materials and other factors. In preferred embodiments, such joining is achieved by welding which is performed using a YAG laser from the outside of the electrode 102, through the electrode wall. The laser joins the cable 182 with the inner surface of the electrode 102. In some embodiments, the weld melts the electrode alloy so that the melt at least partially penetrates the strands of the cable 182 which are touching the inner surface of the electrode 102. It is desirable that little melting of the cable 182 (e.g. strands of DFT) occurs because the strength properties of cobalt-chrome alloy may decrease when it is overheated due to welding.

In preferred embodiments, each electrode 102 is welded to the conductor cable 182 with two welds. The two welds are approximately 0·51-1·02 mm (0·020-0·040 inches) apart along the electrode 102. When stranded cables are used, twisting of the strands between the two welds captures a different set of strands in each weld. After the welding is complete, the strands at the end of the cable 182 are laser fused together by cutting the cable 182 to length near the end of the electrode 102. It may be appreciated that the same methods may be used to weld the cable 182 to the corresponding electrical contact 180.

This welding method ensures that many strands are captured by the welds to connect the cable 182 with the electrode 102 or electrical contact 180 without overheating the cable material. However, it may be appreciated that a single weld may be used. In any case, fusing the end of the cable 182 after welding can increase the load sharing of the strands and the breaking strength of the cable weld. Thus, even those strands that are not directly welded to the electrode 102 or electrical contact 180 can at least partially share the tensile load through the fusing operation.

It may be appreciated that, in some embodiments, at least some of the cables 182 are comprised of a single wire. In such instances, a single weld may be sufficient. In other embodiments, the cables 182 are formed together in a composite cable. Optionally, the cables 182 may be embedded in the wall of the shaft 103.

It may also be appreciated that the electrodes 102 may have other forms. For example, in some embodiments, at least one electrode 102 is comprised of a plurality of elements that are electrically connected to each other. In other embodiments, at least one electrode 102 extends partially around the shaft of the lead 100 so as to impart a directional field. In still other embodiments, at least one electrode has a hollow cylinder shape wherein one or more features are cut from or through its surface. This may allow extension of the length of the electrode without increasing its surface area. Such longer electrodes may reduce the effects of lead migration. Other embodiments include diverse electrode shapes and edge geometries in order to affect the level and variation of current density to optimize the effect of the energy on the target anatomy. It may also be appreciated that at least one electrode 102 may have a composite structure or be comprised of pyrolite carbon which provides for surface geometry increases.

In some embodiments, the lead 100 also includes a tensile element 188, as illustrated in Fig. 24B. The tensile element 188 extends through the space 186 between the stylet tube 174 and the single lumen tube 172. In some embodiments, the tensile element 188 comprises a single strand wire of suitable material, such as cobalt-chrome alloy. In such embodiments, the element 188 typically has a diameter of 0·102 mm (0.004 inches). Optionally, the element 188 may have multiple diameters. For instance, the element 188 may have a larger diameter near the proximal end 105 (such as approximately 0·254 mm (0·010 inches)) and then neck down toward the distal end 101. This may increase the ease of insertion of at least a portion of the proximal end 105 into the implantable pulse generator yet maintain adequate flexibility in the distal end 101 of the lead 100 while retaining adequate tensile strength. It may be appreciated that in some embodiments, more than one tensile element 188 may be used. And, in some embodiments the tensile element 188 is comprised of other materials and forms such as metals, polymers, stainless steel, braids, and cables, to name a few.

The element 188 typically extends from the distal end 101 to the proximal end 105 of the lead 100, however the element 188 may extend any desirable distance. The element 188 is fastened to portions of the lead 100 that allow the element 188 to absorb tensile stress applied to the lead 100 during or after implantation. In particular, the element 188 is tighter or straighter than the conductor cables 182 so as to absorb the tensile load first. Thus, the tensile element 188 is flexible at least near the distal end 101, but has adequate tensile strength (such as greater than or equal to 13·79 kPa (216f)) to guard the cables 182 and welds from breakage. This is preferable to the conductor cables 182 and welds absorbing the tensile load and increases the tensile strength of the lead 100. Such fastening may be achieved with welding, potting, crimping, wrapping, insert molding or any suitable method.

In the embodiment of Fig. 24B, the stylet tube 174, the tensile element 188, and the conductor cables 182 extend through the single lumen tube 172 and are free to move therein. Typically, these components are fixed to the single lumen tube 172 near its proximal and distal ends and the components are unattached therebetween. Thus, as the lead 100 bends or curves during positioning, the stylet tube 174, the tensile element 188, and the conductor cables 182 are each able to move somewhat independently within the single lumen tube 172. Such movement allows greater flexibility in bending and lower applied forces to achieve reduced curve radii in the lead 100. It may be appreciated that the components may be fixed at other locations, allowing freedom of movement therebetween. Likewise, it may be appreciated that the space 186 may optionally be filled with potting material, such as silicone or other material.

In some embodiments, the lead 100 does not include a separate tensile element 188. In such embodiments, the stylet tube 174 may be reinforced with longitudinal wires, strips, coils, embedded braids or other elements to provide additional tensile strength.

As mentioned previously, the distal end 101 of the lead 100 has a closed-end distal tip 106. The distal tip 106 may have a variety of shapes including a ball shape, as shown. The shaped tip provides an atraumatic tip for the lead 100 as well as serving other purposes, such as preventing the distal tip 106 from being withdrawn into the sheath 122. This also serves as an atraumatic tip for the sheath 122. In some embodiments, the diameter of the shaped distal tip 106 is approximately the same as the outer diameter of the sheath 122. For example, in the instance of a ball shaped distal tip 106, if the diameter of the sheath 122 is approximately 1·32-1·45 mm (0·052-0·057 inches), the diameter of the ball may be 1·4-1·52 mm (0·055-0·060 inches). The ball is also sized so as to be passable through the introducing needle 126. However, it may be appreciated that the distal tip 106 may optionally be shaped to allow the lead 100 to be retracted into the sheath 122. For example, the lead 100 and the sheath 122 may have corresponding "keyed" features that allow certain rotations of the lead 100 to pass thru the sheath 122. Or, the lead 100 may include a mechanism which causes the distal tip 106 to be reduced in diameter. Such a mechanism may be actuated at the proximal end of the lead, such as by the stylet 124.

Fig. 24C illustrates a cross-sectional view of an embodiment of a distal tip 106 of a lead 100 having a ball shape, wherein the distal tip 106 is retracted against the distal end of the sheath 122. In this embodiment, the tip 106 is molded from the same material as the shaft 103, such as by a catheter tipping operation. However, it may be appreciated that the ball shape may be formed from any variety of methods and materials, such as silicone, UV adhesives, cyanoacrylates or any suitable material that can be flowed into a shape and cured to maintain the shape. It may also be appreciated that the ball shaped distal tip 106 may also have an additional distal atraumatic feature, such as a silicone tip. Alternatively or in addition, the distal tip 106 may be configured to allow ingrowth of tissue. For example, the distal tip 106 may be comprised of multifilament polymers.

In this embodiment, the tip 106 also includes an internal assembly 200, as illustrated in Fig. 24C. Typically the internal assembly 200 is comprised of metal, such as cobalt-chrome or stainless steel, or other suitable material. The internal assembly 200 acts as a hard barrier that prevents the stylet 124 from protruding out the distal end 100 of the lead 100. In addition, the internal assembly 200 may serve as a mechanism of attaching the stylet tube 174 to the single lumen tube 172. Further, the internal assembly 200 may serve as an anchoring point for the tensile element 188. In addition, when the internal assembly 200 is comprised of a radiopaque material, the assembly may serve as a radiopaque marker under fluoroscopy.

It may be appreciated that in some embodiments the distal tip 106 is not closed-ended. For example, the distal tip 106 may include a passageway to allow pressure relief to aid in inserting or withdrawing the stylet 124. Likewise, it may be appreciated that in some embodiments, the distal tip 106 does not include an internal assembly 200. In such embodiments, the stylet tube 174 may be attached to the single lumen tube 172 by potting or other mechanisms.

In some embodiments, the lead 100 includes potting 190 between the stylet tube 174 and the single lumen tube 172, as illustrated in Fig. 24C. Examples of such potting 190 include silicone, other polymers, adhesive, or melting of the material which forms the single lumen tube 172. Potting 190 may be disposed along the distal end 101, the proximal end 105 or along the length of the lead 100. In some instances, the potting 190 provides additional resistance to failure due to such factors as electrical shorting or weld breakage. Potting can also prevent migration of bodily fluids through portions of the lead. Potting can also improve ease of insertion of the proximal end of the lead into the pulse generator.

In some embodiments, the distal end of the lead 100 is overmolded or cast with polymer or other suitable material to encapsulate the components, with the exception of the outer surface of the electrodes.

Fig. 24D provides a side cut-away view a portion of the distal end 101 of the lead 100 of Fig. 24A. An electrode 102 is shown wrapped around the single lumen tube 172, and a conductor cable 182, having a stripped end, is attached to the electrode 102. The stylet tube 174 is shown extending through the single lumen tube 172. Likewise, the tensile element 188 is shown extending alongside the stylet tube 174.

In some embodiments, the lead 100 includes a proximal end cap 200, such as illustrated in Fig. 24E. The proximal end cap 200 is disposed on the proximal end 105 of the lead 100, as illustrated in Fig. 24A. In this embodiment, the end cap 200 comprises a clamping ring 202 and a hollow shaft 204, wherein the shaft 103 of the lead 100 extends over the hollow shaft 204 and abuts the clamping ring 202. The shaft 103 is attached to the end cap 200 by suitable mechanisms, such as by adhesive, melting of the shaft material, overmolding or by clamping with an external ring, to name a few. The end cap 200 also includes a lumen 206 which connects with the stylet lumen 104 of the lead 100. Thus, the stylet 124 is insertable through the lumen 206 and advanceable therethrough. In some embodiments, the opening to the lumen 206 is beveled, as shown, to assist in such insertion.

The clamping ring 202 provides a solid point against which an implantable pulse generator connector block set screw may fixate, holding the lead 100 in place within the header of the pulse generator. The end cap 200 may also serve as an anchoring point for the tensile element 188. Likewise, the end cap 200 may be used to connect stylet tube 174 and the single lumen tube 172 together. Typically, the end cap 200 is comprised of a metal, such as cobalt-chrome or stainless steel. However, the end cap 200 may alternatively be comprised of a polymer, optionally with an embedded strength member along the clamping ring 202 to resist the force of the set screw. In some embodiments, the end cap 200 is used as an electrode. In such embodiments, the end cap 200 is connected with a conductor cable 182 and an electrode 102 disposed on the distal end of the lead 100.

### Stylet

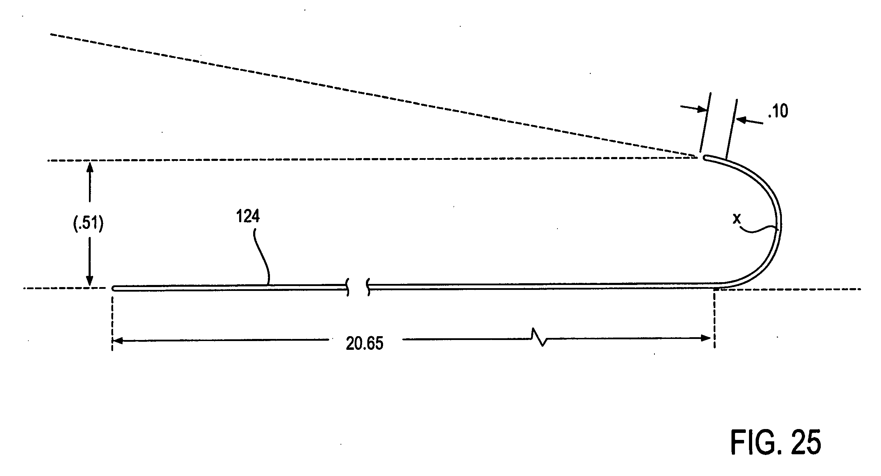

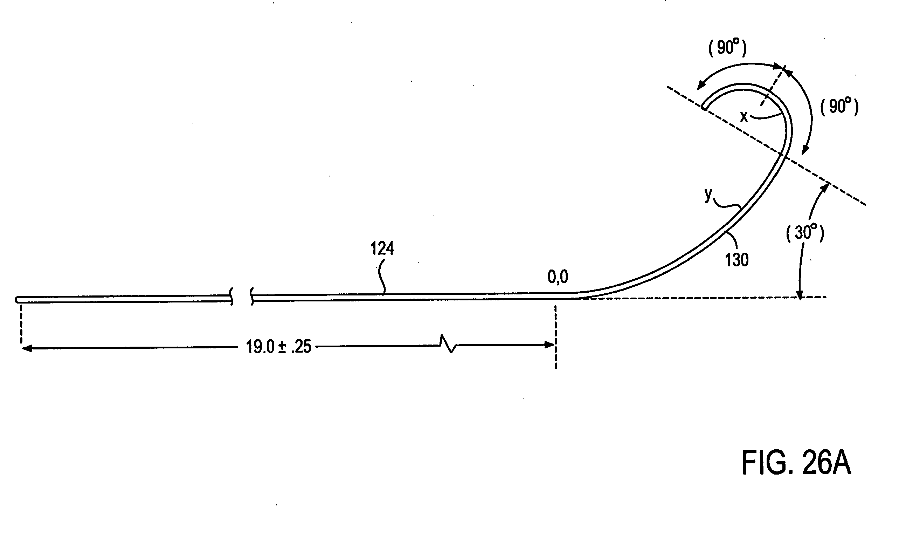

Figs. 25, 26A-26B illustrate embodiments of a stylet 124 of the present system. In some embodiments, the stylet 124 is comprised of superelastic nitinol. Nitinol is biocompatible and provides a variety of desirable features. For example, the nitinol material is elastic enough to allow the stylet 124 to straighten when inserted into a lead 100 and captured within a straight portion of the sheath 122. However, it is able to recover its shape once the lead 100 is advanced past the distal end 128 of the sheath 122, at which point the stylet 124 has enough bending stiffness to force the distal end 101 of the lead 100 into a desired curve for delivery. In particular, it forces the distal end 101 of the lead 100 to curve around toward the target DRG along the nerve root angulation. This allows the lead 100 to be successfully steered to position at least one of the electrodes 102 on, near or about the target DRG, particularly by making a sharp turn along the angle θ of Fig. 7.

Typically, the stylet 124 has a diameter in the range of approximately 0.2-0.61 mm (0.008-0.024 inches), preferably approximately 0.2-0.46 mm (0.008-0.018 inches). In some embodiments, the stylet 124 has a diameter of 0.254 mm (0.010 inches), particularly when used with a lead 100 having an outer diameter of 1.02 mm (0.040 inches). Superelastic nitinol, especially in the 0.254 mm (0.010 inch) diameter range, has relatively low stiffness which is beneficial for atraumatic guidance of the stylet 124/lead 100 combination near nerve and other tissue. Thus, the stylet 124/lead 100 combination may tend to be guided between anatomical layers rather than be forced through tissue.

Typically, the stylet 124 has a length that is approximately 1 cm longer than the lead 100. In addition, the distal end 130 of the stylet 124 is preset into a curve. Fig. 25 illustrates a stylet 124 having a primary curve X. The primary curve X may be described in terms of an arch shape or half circle having a perimeter along which the stylet 124 extends. Thus, a 180 degree primary curve X would be comprised of the distal end of the stylet 124 extending along the entire half circle. A 90 degree primary curve X would be comprised of the distal end of the stylet 124 extending half way around the half circle. The primary curve X may be formed by the stylet 124 extending up to 360 degrees, typically up to 270 degrees, more typically up to 180 degrees. The embodiment of Fig. 25 illustrates a primary curve X formed by the distal end of the stylet 124 extending 170 degrees along the perimeter of the half circle, wherein the half circle has a radius of 6.35 mm (0.25 inches). In this embodiment, the distal tip of the stylet 124 has a 2.54 mm (0.10 inch) straight section.

In some embodiments, the curve is comprised of a primary curve X and a secondary curve Y. The embodiment of Fig. 26A illustrates a primary curve X formed by the distal end of the stylet 124 extending 180 degrees along the perimeter of the half circle, wherein the half circle has a radius of 6.35 mm (0.25 inches). In this embodiment, stylet 124 also has a secondary curve Y which is proximal and adjacent to the primary curve X. It may be appreciated, however, that no secondary curve Y may be present (as in Fig. 25) or the secondary curve Y may be formed at any location along the stylet 124 and may not be adjacent to the primary curve X. Typically, the secondary curve Y has a larger radius of curvature than the primary curve X. In this embodiment, the secondary curve Y has a radius of curvature of 3.81 cm (1.5 inches).

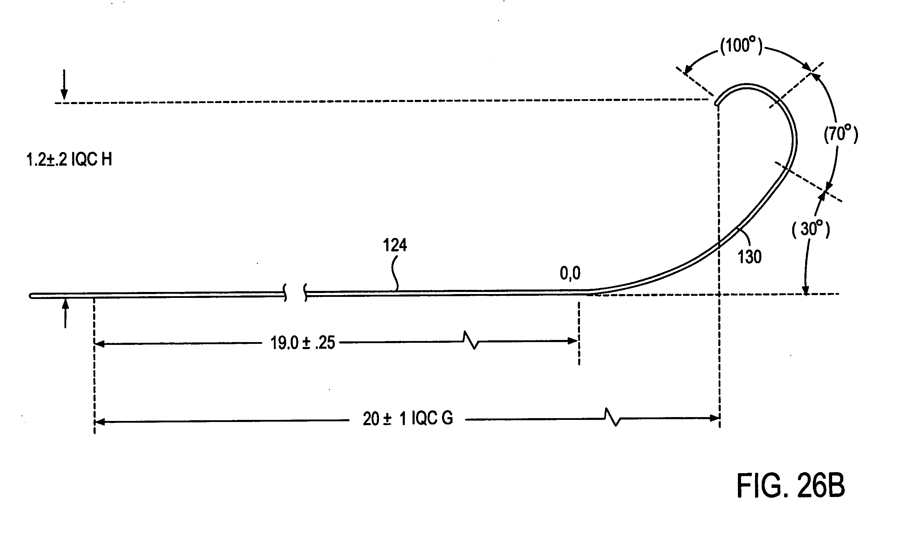

In other embodiments, the primary curve X and/or secondary curve Y are compound curves. A compound curve is comprised of two or more subcurves. For example, Fig. 26B
illustrates primary curve X comprised of a subcurve having a radius of curvature of 6.35 mm (0.25 inches) and another subcurve having a radius of curvature of 9·4 mm (0·37 inches). Such compound curvatures allow for greater variety of overall shape. In this example, the compound curvature creates a slightly wider primary curve X.

Overall, the primary curve X may be considered "U" shaped. It may be appreciated, that other curve shapes may be used to increase ease of delivery or increase anchoring of the lead 100 about the desired anatomy. Examples include a "V" shape, an "S" shape or a coil, to name a few.

It may be appreciated that the stylet 124 is formed from other materials in other embodiments, such as metals, alloys, polymers and stainless steel. Stainless steel may be preferred with the lead 100 is to be delivered in a relatively straight or straighter configuration. The stylet 124 may be supplied in a straight or pre-bent configuration. Likewise, the stylet 124 may be bent by the practitioner prior to insertion into the lead 100.

In some embodiments, the distal tip of the stylet 124 is rounded or otherwise formed to resist embedding into the wall of the stylet tube 174 or damaging the stylet tube 174 during insertion. Likewise, in some embodiments, the stylet 124 is coated with polytetrafluoroethylene (PTFE), parylene or other coating material to increase lubricity. This eases insertion of the stylet 124 through the stylet tube 174 and resists jamming or hangups. In addition, a lubricious coating can increase tactile feedback of the stylet motion within the lead 100. As mentioned above, such lubricity may be provided by the stylet tube 174 itself or a coating along the stylet lumen 176, however such coating of the stylet 124 may be used alternatively or in addition.

In some embodiments, the stylet 124 includes a gripping device near its proximal end. The gripping device allows the stylet 124 to be more easily or more ergonomically grasped for torquing the stylet 124. Such torquing changes the steering direction of the distal end 130 of the stylet 124. The gripping device may be fixedly or removably attached to the stylet 124. In some embodiments, the gripping device also indicates the direction of the curvature of the distal end 130.

It may be appreciated that more than one stylet 124 may be inserted into a lead 100, particularly into the same stylet tube 174. In such instances, each stylet 124 may have a different bend geometry or stiffness and manipulation of the stylets 124 could allow for increased steering capability. Likewise, the lead 100 may include more than one stylet tube 174 or insertion of multiple stylets 174.

Alternatively or in addition, the shape and stiffness of the stylet 124 may be actively controlled with the use of control wires or other similar devices. For instance, in some embodiments, the stylet 124 has a tubular shape and features are cut partially through the tube diameter near the distal end of the stylet 124. This allows the tube to elastically bend in the region of the cut features. A thin wire is attached to an inner wall of the tubular stylet 124 distally of the cut features and extends through the proximal end of the tube to an actuating handle. When the wire is put under tension, the distal end of the stylet 124 bends from its original shape. When the tension is removed, the distal end of the stylet 124 recovers to its original shape. More than one wire can be used for multiple bends. Such a stylet 124 may be comprised of any suitable materials, particularly superelastic nitinol.

It may be appreciated that in some embodiments, the stylet 124 is fixedly attached or embedded in the lead 100. In such instances, the stylet 124 serves to maintain curvature, steerability and stiffness to the lead 100 and is not removed.

It may further be appreciated that the lead 100, stylet 124 or sheath 122 may alternatively be manipulated by active steering control elements. Such control elements would be managed by external controls.

It may further be appreciated that the stylet 124 and/or sheath 122 may have a substantially straight configuration. Such a straight configuration may be particularly useful when making larger bends, such as 90 degree bends. For example, if the nerve root angulation of the target DRG is relatively large, a straight sheath 122 may be used to position the lead 100 near the nerve root wherein the curved stylet 124 allows the lead 100 to bend along the nerve root angulation upon exiting the sheath 122. Thus, together the sheath 122 and stylet 124 form an approximately 90 degree bend. Or, the curvature of the sheath 122 itself may be sufficient to direct the lead 100 toward the target DRG. In such instances, a straight stylet 124 may be used or the lead 100 may be advanced without the use of a stylet.

### Shapeable Sheath Embodiments

In some embodiments, the sheath 122 is comprised of a shapeable material. Such a material is shapeable by simply bending the material, wherein the material substantially maintains the bent shape. In preferred embodiments, the shapeable material comprises polyimide with stainless steel wires embedded therein. The wires assist in providing strength and shapeability. In some embodiments, the wires are embedded axially therein. Any number of wires may be present, such as one, two, three, four, six, eight or more. In some embodiments, four wires are present. The wires may have a variety of thicknesses. Example materials include 470-VII.5 PTFE ID/BRAID w/4 AXIAL supplied by MicroLumen (Tampa, FL). In other embodiments, one or more wires are embedded in a coiled configuration.

Typically, sheaths 122 comprised of a shapeable material have the same features as the sheaths 122 described above and are used with the other delivery devices in the same manner. However, the shapeable sheath does not rely on a preformed or preset bend near its distal end. Rather, the shapeable sheath can be bent to any angle α at the time of use. Likewise, the shapeable sheath can be rebent and readjusted as many times as desired. This allows the practitioner to adjust the angle α as needed before or during a procedure to more desirably access the target anatomy. This may be particularly useful in patients that have irregular anatomy or unanticipated anatomical features, such as due to progressive disease.

### Multiple Sheath Embodiments

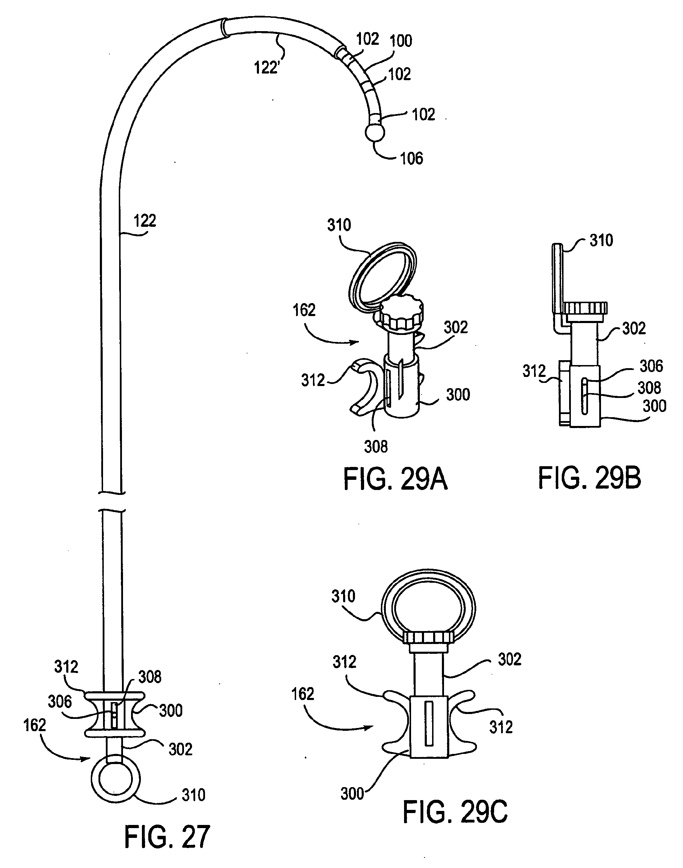

It may also be appreciated that multiple sheaths may be used to desirably direct the lead 100 toward a target anatomy, such as a target DRG. For example, as illustrated in Fig. 27, an additional sheath 122' may be used with the above described delivery system 120. In such situations, the additional sheath 122' is advanceable through sheath 122, and the lead 100 is advanceable through the additional sheath 122'. The additional sheath 122' may have any desired curvature or may be substantially straight. Each of the sheath 122, the additional sheath 122' and the lead 100 may be advanced and retracted in relation to each other. In some embodiments, the sheaths are moveable so that its distal end of the additional sheath 122' extends approximately 12-20mm, typically approximately 15mm, beyond the distal end of the sheath 122. Such movements, in combination with the shapes (e.g. curvatures) of the delivery devices, provide increased maneuverability and variety in the angles through which the devices may be advanced. In addition, the multiple sheath design increases the ability to impart lateral forces, such as toward a foramen, particularly at substantial distances from the entry point to the epidural space. As described above, the delivery system 120 may be used to target anatomy at multiple spinal levels above or below the insertion point of the needle. The greater the distance from the insertion point, the ability to impart lateral forces with the sheath 122 becomes more challenging. In some instances, a foramen may be at least partially stenosed, creating difficulty in advancing a lead therein. It may be desired to impart lateral forces with the sheath122 to access this type of foramen. The additional sheath 122' provides additional stiffness, steerability, and length which can be helpful in such access.

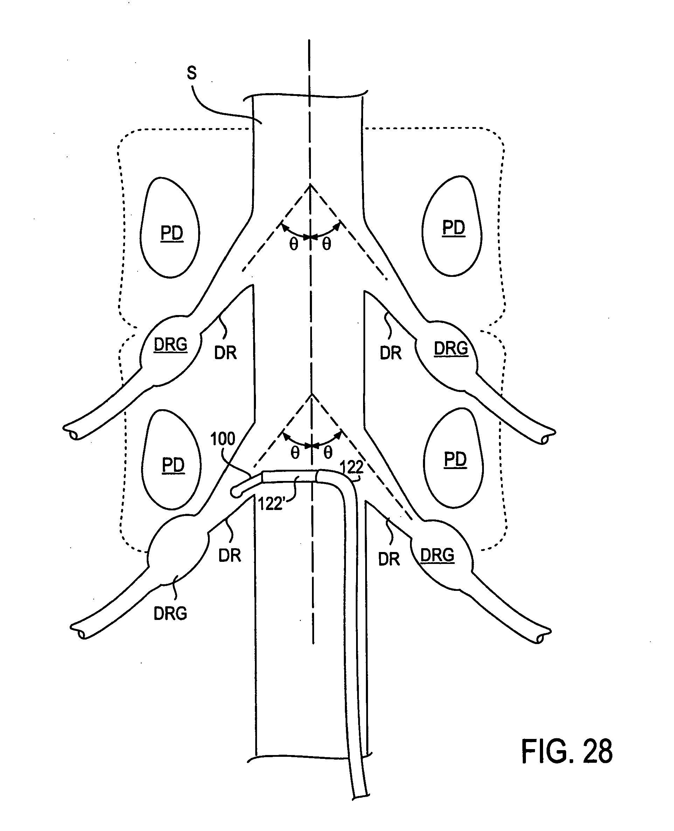

In some embodiments, the additional sheath 122' has a substantially straight configuration. A straight configuration may be used to traverse greater expanses than with the sheath 122 alone. For example, in some patients and/or in some portions of the anatomy (such as in the sacrum or when advancing through the sacral hiatus) the epidural space may be particularly wide. Or, the sheath 122 may be positioned within the epidural space between the midline and the "gutter" of the epidural space opposite the target DRG, as illustrated in Fig. 28, so that a larger portion of the epidural space is to be traversed. Traversing these greater expanses may be more easily achieved with the use of an additional sheath 122'. In such embodiments, the additional sheath 122' has a stiffness that allows for transverse translation. Advancement or translation of the sheath 122' may be achieved with the use of, for example, a sliding mechanism disposed within the hub 162. The lead 100 is then advanceable through the additional sheath 122', such as described above, to a position so as to stimulate the target DRG.

In one embodiment, the sheath 122 has an outer diameter of approximately 0.063 inches, an inner diameter of approximately 1·45 mm (0·057 inches), and a working length of approximately 30 cm. In this embodiment, the additional sheath 122' has an outer diameter of approximately 1·32 mm (0.052 inches), an inner diameter of approximately 1·17 mm (0.046 inches) and a working length of approximately 45cm. Together, the sheaths 122, 122' are passable through a 14 gauge needle having an inner diameter of approximately 1·7 mm (0·067 inches).

In some embodiments, the sheath 122 has a curvature and the additional sheath 122' also has a curvature. In such instances, retraction of the additional sheath 122' within the sheath 122 may cause the two curvatures to wedge together. This may be prevented by restricting the distance the additional sheath 122 may be retracted within the sheath 122. In some embodiments, this is achieved by a control hub 162 having a sliding mechanism and a limiter. Figs. 29A, 29B, 29C illustrate a perspective view, a side view and a front view, respectively, of an embodiment of such a control hub 162. Here, the hub 162 includes a base 300 and a slidable extender 302. The base 300 attaches to the proximal end of the sheath 122 and the slidable extender 302 attaches to the proximal end of the additional sheath 122'. Advancement and retraction of the extender 302 in relation to the base 300, moves the additional sheath 122' in relation to the sheath 122. Such advancement and retraction is limited by a limiter. In this embodiment, the limiter comprises a protrusion 306 along the extender 302 which protrudes into a slot 308 along the base 300. The protrusion 306 slides along the slot 308 as the extender 302 moves, limited at each end by the confines of the slot 308. Thus, in this embodiment, advancement is limited in addition to retraction. Such limitation of advancement may be used when a predetermined or known distance of advancement is desired. In some embodiments, the predetermined distance is 15mm. Likewise, when repeated advancement and retraction is desired, such as to penetrated through an obstruction, the use of the limiter 304 may be desired. This allows quick, repeatable movements through a known distance without risk of over-advancement or over-retraction. In some embodiments, the hub 162 includes ergonomic handles to assist in manipulation. For example, in one embodiment, the extender 302 includes a ring 310 and the base 300 includes hooks 312. Insertion of fingers under the hooks 312 and a thumb through the ring 310 allows easy one-handed manipulation of the hub 162 by moving the thumb toward and away from the fingers of the hand.

### Other Embodiments

In some instances, the lead 100 has a preset curvature which assists in directing the lead 100 to the target anatomy upon advancement.

The lead 100 and the stylet 124 may both have a curvature.

Any number of seaths may be used with any combination of curvatures. Likewise, each combination can be used with curved stylets or curved or straight leads.

The desired combination of devices and curvatures may depend on a variety of factors, including the approach used (such as antegrade, retrograde or contralateral), the choice of target anatomy, and the particular anatomical features of the individual patient, to name a few.

It may further be appreciated that when a delivery device is described as directing the lead toward a target anatomy, such direction may be in the general vicinity of the target anatomy allowing for additional steps to direct the lead even closer toward the target anatomy. For example, a curved sheath may direct the lead away from the midline of the spinal column, toward a target DRG. However, straight advancement of the lead therefrom may be below, above or not desirably close enough to the target DRG. Therefore, additional directing of the lead toward the target DRG may be desired to position the lead closer to the target DRG. The curved stylet may be used to direct the lead again toward the target DRG, such as along a nerve root sleeve angulation. Such steps may optimize positioning of the lead.

### Connection to Implantable Pulse Generator

As mentioned above, the proximal ends of the leads 100 are connected with an IPG which is typically implanted nearby, such as along the back, buttock or abdomen. The IPG may be any conventional IPG which provides stimulation signals to the one or more leads. Or, the IPG may be particularly adapted for targeted treatment of the desired anatomies. For example, the delivery devices of the present invention may be used in combination with the implantable stimulation system described in U.S. Patent Publication No. 2010-0137938, "Selective Stimulation Systems and Signal Parameters for Medical Conditions" filled October 27, 2009. Such targeted treatment minimizes deleterious side effects, such as undesired motor responses or undesired stimulation of unaffected body regions. This is achieved by directly neuromodulating a target anatomy associated with the condition while minimizing or excluding undesired neuromodulation of other anatomies. In some embodiments, the lead and electrode(s) are sized and configured so that the electrode(s) are able to minimize or exclude undesired stimulation of other anatomies. In other embodiments, the stimulation signal or other aspects are configured so as to minimize or exclude undesired stimulation of other anatomies.

### Strain Relief Support for Lead Connection to IPG

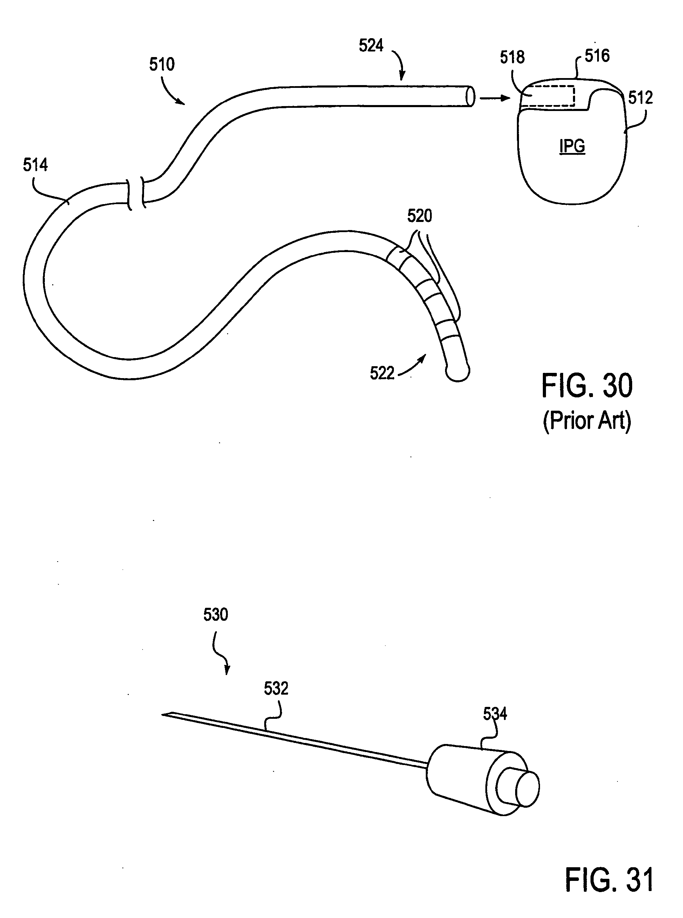

Typically, the IPG is surgically implanted under the skin at a location that is remote from the stimulation site. The leads are tunneled through the body and connected with the IPG to provide the stimulation pulses. Fig. 30 illustrates a conventional stimulation system 510 used to stimulate tissues or organs within the body. The system 510 includes an IPG 512 and at least one lead 514. The IPG 512 includes a header 516 having at least one connection port 518 for electrically connecting with the lead 514. The lead 514 includes at least one electrode 520, typically disposed near its distal end 522, and a conductive wire extending from each electrode 520 to its proximal end 524. The proximal end 524 of the lead 514 is inserted into the connection port 518 to electrically connect the conductive wire with the electronic circuitry within the IPG 512.

The leads are generally of a fragile nature and care must be taken to minimize strain on the leads during implantation and throughout the life of the device. To reduce strain on the lead, the lead is often implanted in a looped configuration and sutured in place. In this manner, strain put on the lead may be absorbed by the looped coil. However, this practice involves additional manipulation of the fragile lead and a larger implantation area to accommodate the looped configuration.

In addition, a particularly vulnerable portion of the lead is the point of connection with the IPG. It is typically desired that the lead be soft and "floppy" so as to conform to bends in the anatomy along its path. In contrast, the IPG is typically a rigid body configured to withstand encapsulation and tissue contraction. To connect the lead to the IPG a portion of the lead is inserted into the IPG and fixed in place. Thus, as the lead exits the IPG the lead endures an abrupt transition from fully supported by the IPG to fully unsupported. This portion of the lead is vulnerable to kinking, strain and damage. In addition, the soft and floppy characteristics of the lead may also prove challenging when trying to insert the lead into the IPG.

Thus, it is desired to provide devices, systems and methods for improving handling of the lead, including insertion of the lead into an IPG, and reducing any vulnerability of the lead in the area of connection to the IPG. At least some of these objectives will be met by the present invention.

Devices, systems and methods are provided to improve connectability of a lead, such as a conventional lead or any of the leads of the present invention described herein, to an IPG and to reduce any vulnerabilities of this connection. As mentioned above, the soft and floppy characteristics of many leads may provide both handling issues and longevity issues when connected with an IPG. For example, insertion of a floppy lead into an IPG may be difficult and time consuming. And, the portion of the lead exiting the IPG may be vulnerable to kinking, strain and damage. The present invention assists in overcoming these issues by providing a strain relief support which is joinable with the proximal end of a lead. The support provides rigidity to the proximal end of the lead to assist in handling and insertion of the proximal end of the lead into an IPG. And the support protects the lead from possible vulnerabilities near the connection point with the IPG.

Fig. 31 illustrates an embodiment of a strain relief support 530. The support 530 comprises a support member 532 and a detachable hub 534. In this embodiment, the support member 532 comprises an elongate shaft sized to be inserted into the proximal end 524 of a lead 514. Typically, the lead includes a plurality of lumens, such as a separate lumen for each conductive wire. Additionally, the lead may include a stylet lumen or other lumen. The support member 532 is typically inserted into the stylet lumen or other lumen so as to internally support the proximal end of the lead. However, it may be appreciated that the support member 532 may be inserted into any lumen or be attached to an outer surface of the lead. The hub 534 is attachable to the support member 532 to provide a handle or gripping structure to assist in manipulating the support member 532.

The strain relief support 530 may be comprised of any suitable materials including metals (such as stainless steel, nitinol, MP35N, etc.) or plastics (such as nylon, polycarbonate, polyurethane, etc.).

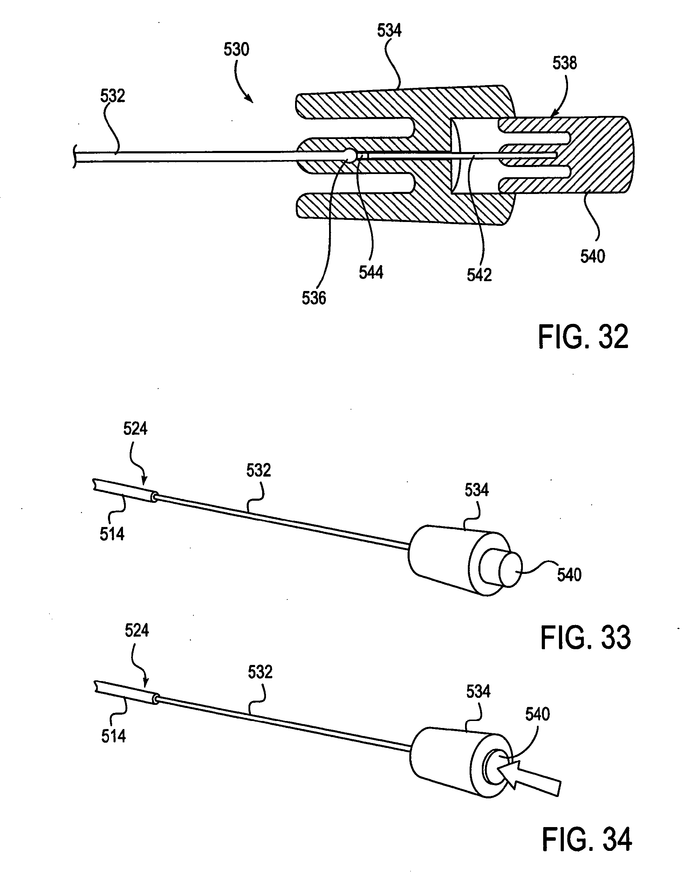

Fig. 32 illustrates a cross-section of the strain relief support 530, including the support member 532 and the hub 534. As shown, the support member 532 has an end structure 536 which is disposed within the hub 534. The hub 534 includes a plunger 538 comprising a plunger button 540 attached to a plunger shaft 542. The plunger shaft 542 extends through a channel 544 in the hub 534 toward the end structure 536 of the support member 532. Depression of the plunger button 540 translates the plunger shaft 542 through the channel 544 so that the plunger shaft 542 contacts the end structure 536. Continued depression applies force to the end structure 536 and pushes the end structure 536 out of the hub 534 as the hub material flexes to allow such movement. The support member 532 is thus released and the hub 34 is considered detached. It may be appreciated that the hub 534 may alternatively be detached by other mechanisms, such as by break-away from a friction fit with the support member 532.

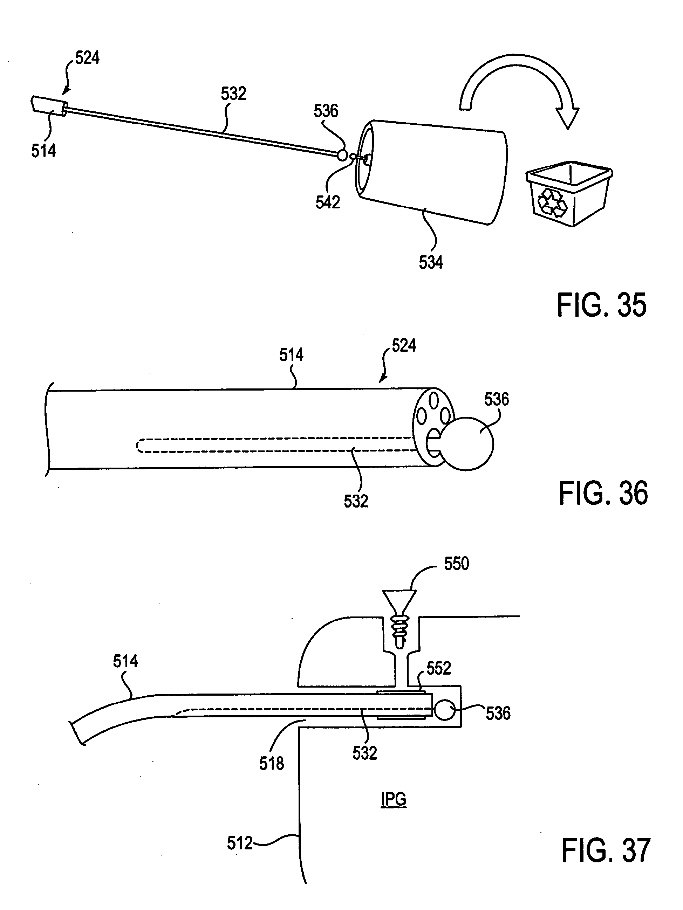

Figs. 33-35 illustrate insertion of the support member 32 into the proximal end 524 of a lead 514. Once the proximal end 524 of the lead 514 has been tunneled to a pocket location within the patient's body and the pocket is ready to accept the IPG, the proximal end 524 is ready to receive the support member 532. Typically, the support member 532 is pre-attached to the hub 534 to allow easy grasping by the user. The user holds the hub 534 and directs the support member 532 into the proximal end 524 of the lead 514, as shown in Fig. 33. In particular, the support member 532 is inserted into a desired lumen in the lead 514. The hub 534 is then detached from the support member 532 by depression of the plunger button 540, as illustrated in Fig. 34. Fig. 35 provides a side view of Fig. 34. As shown, the plunger shaft 542 has pushed the end structure 536 out of the hub 534 so that the hub 534 is detached and can be disposed or recycled. The support member 532 can be further inserted into the lead 514 so that the end structure 536 abuts the lead 514, as shown in Fig. 36. Such insertion can be achieved by pushing the end structure 536, such as with a finger or tool, until the end structure 536 is desirably positioned. Typically, the end structure 536 is sized to be larger than the lumen into which the support member 532 is being inserted so as to remain outside of the lead 514. This resists distal migration of the support member 532 and allows for easy removal of the support member 532 from the proximal end 524 if desired. In this embodiment, the end structure 536 has a round or ball shape, however it may be appreciated that the structure 536 may have any suitable shape, particularly a shape which is easily graspable and resists insertion into the lead 514. However, it may be appreciated that the end structure 536 may optionally be sized and shaped to be inserted into the proximal end 524 of the lead 514 if desired.

The proximal end 524 of the lead 514 may then be inserted into the connection port 518 of the IPG 512, as illustrated in Fig. 37. The support member 532 provides rigidity to the proximal end 524 of the lead 514 to assist in handling during insertion of the proximal end 524 into the connection port 518. The proximal end 524 may then be fixed within the connection port 518 with the use of a set screw 550. The set screw 550 is advanced toward the lead 514 and tightened against the lead 514 to hold the lead 514 within the connection port 518 by frictional force. In some embodiments, the lead 514 includes a cuff 552 aligned to contact the set screw 550. The cuff may be comprised of any suitable material, such as MP35N (CoCr). Such fixation of the support member 532 within the connection port 518 also resists dislodgement of the support member 532 and possible migration.

As shown, the support member 532 extends beyond the IPG 512 so that the lead 514 is supported outside of the IPG 512. This diminishes the abrupt transition from fully supported by the IPG 512 to fully unsupported. Consequently, this portion of the lead is less vulnerable to kinking, strain and damage. It may be appreciated that the support member 532 may be tapered toward its distal end to gradually reduce stiffness along the lead 514 as the lead 514 exits the connection port 518.

The above described embodiment shows the support member 532 having a straight shape. It may be appreciated that the support member 532 may alternatively have a curved, bent, folded, compound shape or other shape.

### Applications

It may be appreciated that the systems of the present invention may be used or adapted for use in stimulating other neural targets or other tissues throughout the body. Some examples include occipital nerves, peripheral nerve branches, nerves in the high cervical area, nerves in the thoracic area, and nerves in the lower sacral area.

A variety of pain-related conditions are treatable with the systems of the present invention. In particular, the following conditions may be treated:
1) Failed Back Surgery syndrome
2) Chronic Intractable Low Back Pain due to:
   A) Unknown Etiology
   B) Lumbar facet disease as evidenced by diagnostic block(s)
   C) Sacroiliac Joint disease as evidenced by diagnostic block(s)
   D) Spinal Stenosis
   E) Nerve root impingement - non-surgical candidates
   F) Discogenic Pain - discography based or not
3) Complex Regional Pain Syndrome
4) Post-Herpetic Neuralgia
5) Diabetic Neuropathic Pain
6) Intractable Painful Peripheral Vascular Disease
7) Raynaud's Phenomenon
8) Phantom Limb Pain
9) Generalized Deafferentation Pain Conditions
10) Chronic, Intractable Angina
11) Cervicogenic Headache
12) Various Visceral Pains (pancreatitis, etc.)
13) Post-Mastectomy Pain
14) Vulvodynia
15) Orchodynia
16) Painful Autoimmune Disorders
17) Post-Stroke Pain with limited painful distribution
18) Repeated, localized sickle cell crisis
19) Lumbar Radiculopathy
20) Thoracic Radiculopathy
21) Cervical Radiculopathy
22) Cervical axial neck pain, "whiplash"
23) Multiple Sclerosis with limited pain distribution

Each of the above listed conditions is typically associated with one or more DRGs wherein stimulation of the associated DRGs provides treatment or management of the condition.

Likewise, the following non-painful indications or conditions are also treatable with the systems of the present invention:
1) Parkinson's Disease
2) Multiple Sclerosis
3) Demylenating Movement Disorders
4) Physical and Occupational Therapy Assisted Neurostimulation
5) Spinal Cord Injury - Neuroregeneration Assisted Therapy
6) Asthma
7) Chronic Heart Failure
8) Obesity
9) Stroke - such as Acute Ischemia
Again, each of the above listed conditions is typically associated with one or more DRGs wherein stimulation of the associated DRGs provides treatment or therapy. In some instances, Neuroregeneration Assisted Therapy for spinal cord injury also involves stimulation of the spinal column.

It may be appreciated that the systems of the present invention may alternatively or additionally be used to stimulate ganglia or nerve tissue. In such instances, the condition to be treated is associated with the ganglia or nerve tissue so that such stimulation provides effective therapy. The following is a list of conditions or indications with its associated ganglia or nerve tissue:
1) Trigeminal Neuralgia (Trigeminal Ganglion)
2) Hypertension (Carotid Sinus Nerve / Glossopharangyl Nerve)
3) Facial Pain (Gasserian Ganglion)
4) Arm Pain (Stellate Ganglion)
5) Sympathetic Associated Functions (Sympathetic Chain Ganglion)
6) Headache (Pterygoplatine Ganglion/Sphenopalatine Ganglion)

It may also be appreciated that the systems and devices of the present invention may also be used to stimulate various other nerve tissue including nerve tissue of the peripheral nervous system, somatic nervous system, autonomic nervous system, sympathetic nervous system, and parasympathetic nervous system, to name a few. Various features of the present invention may be particularly suited for stimulation of portions of these nervous systems. It may further be appreciated that the systems and devices of the present invention may be used to stimulate other tissues, such as organs, skin, muscle, etc.

## Claims

1. A system for accessing a nerve root which extends from a spinal column along a nerve root sleeve angulation, the system comprising:
a lead (100) comprising a shaft (103) having a stylet lumen (104) extending at least partially therethrough and at least one electrode (102) disposed thereon;
a sheath (122) having a pre-curved distal end (128), wherein the sheath is configured to extend over the shaft of the lead; and
a stylet (124) having a pre-curved distal end (130), wherein the stylet is configured to be positioned within the stylet lumpen of the lead,
wherein the lead is configured to be positioned along the spinal column, wherein positioning of the pre-curved distal end of the sheath over the lead bends the lead along a first curvature and wherein advancement of the lead and the stylet therein beyond the sheath bends the lead along a second curvature.

2. A system as in claim 1, wherein the sheath (122) is configured to be advanced through an introducing needle (126) configured to access an epidural space of the spinal column.

3. A system as in claim 2, wherein the introducing needle (126) has an inner diameter of less than or equal to 1.7 mm (0.067 inches).

4. A system as in claim 1, wherein the distal end (128) of the sheath (122) is pre-curved having an angle in the range of 15 to 165 degrees.

5. A system as in claim 4, wherein the distal end (128) of the sheath (122) is pre-curved having an angle in the range of 80 to 165 degrees.

6. A system as in claim 1, wherein the distal end (130) of the stylet (124) is pre-curved having a primary curve and a secondary curve.

7. A system as in claim 6, wherein the primary curve has an arch shape of 180 degrees.

8. A system as in claim 6, wherein the secondary curve is proximal and adjacent to the primary curve.

9. A system as in claim 6, wherein the secondary curve has a larger radius of curvature than the primary curve.

10. A system as in claim 6, wherein the primary curve and/or the secondary curve are compound curves.

11. A system as in claim 1, wherein the lead (100) has a closed-end distal tip (106) having a shape which resists advancement of the sheath (122) over the distal tip.

12. A system as in claim 11, wherein the shape comprises a ball shape.

13. A system as in claim 11, wherein the distal tip (106) of the lead (100) provides an atraumatic cover for the distal end of the sheath (122).

14. A system as in claim 1, wherein the sheath (122) has a stiffness which allows advancement along an epidural space of the spinal column to a position wherein the pre-curved distal end (128) of the sheath can direct the lead toward the nerve root.

15. A system as in claim 1, wherein the sheath (122) has a minimum stiffness of 4.48 kPa (0.65 lbs·in²).

16. A system as in claim 1, wherein the sheath (122) is comprised of a material selected from polyimide and polyetheretherketone.

17. A system as in claim 1, further comprising an additional sheath (122') having a distal end, wherein the additional sheath is configured to pass within the sheath (122) so that its distal end extends beyond the curved distal end (128) of the sheath.

18. A system as in claim 17, wherein the distal end of the additional sheath (122') is curved so that positioning the pre-curved distal end (128) of the sheath (122) over the lead (100) bends the lead along the first curvature and wherein advancement of the curved distal end of the additional sheath beyond the pre-curved distal end of the sheath bends the lead along a second curvature.

19. A system as in claim 17, wherein the distal end of the additional sheath (122') is substantially straight so that positioning the pre-curved distal end (128) of the sheath (122) over the lead (100) bends the lead along the first curvature and wherein advancement of the distal end of the additional sheath beyond the pre-curved distal end of the sheath directs the lead in a substantially straight direction.

20. A system as in claim 1, wherein the lead (100) is sized to fill an inner diameter of the sheath (122) so as to resist kinking of the sheath.

21. A system as in claim 1, wherein the sheath (122) is comprised of a thermoset material.

22. A system as in claim 1, wherein the sheath (122) is comprised of a unidurometer material.

23. A system as in claim 1, wherein the sheath (122) has stiffness transitions.

24. A system as in claim 1, wherein the sheath (122) is comprised of a metal/polymer construct.

25. A system as in claim 1, wherein the sheath (122) is at least partially radiopaque.

26. A system as in claim 25, wherein the sheath (122) is loaded with radiopaque material or wherein the sheath includes at least one radiopaque marker.

## Patentansprüche

1. Vorrichtung für den Zugriff auf eine Nervenwurzel, die sich von einer Wirbelsäule entlang einer Angulierung der Nervenwurzeltasche erstreckt, wobei die Vorrichtung Folgendes aufweist:
eine Leitung (100), die einen Schaft (103) mit einem Mandrinlumen (104), welches sich mindestens teilweise dort hindurch erstreckt, und mindestens eine Elektrode (102), welche daran angeordnet ist, aufweist;
eine Ummantelung (122) mit einem vorgekrümmten distalen Ende (128), wobei die Ummantelung konfiguriert ist, sich über den Schaft der Leitung zu erstrecken; und
ein Mandrin (124) mit einem vorgekrümmten distalen Ende (130), wobei der Mandrin für die Positionierung im Innern des Mandrinlumens der Leitung konfiguriert ist,
wobei die Leitung für die Positionierung entlang der Wirbelsäule konfiguriert ist, wobei die Positionierung von dem vorgekrümmten distalen Ende der Ummantelung über der Leitung die Leitung entlang einer ersten Krümmung biegt und wobei das Vorschieben der Leitung und des Mandrins darin über die Ummantelung hinaus die Leitung entlang einer zweiten Krümmung biegt.

2. Vorrichtung nach Anspruch 1, wobei die Ummantelung (122) konfiguriert ist, durch eine Einführungsnadel (126), welche für den Zugang zu einem Epiduralraum der Wirbelsäule konfiguriert ist, vorgeschoben zu werden.

3. Vorrichtung nach Anspruch 2, wobei die Einführungsnadel (126) einen Innendurchmesser von weniger als oder gleich 1,7 mm (0,067 Inch) aufweist.

4. Vorrichtung nach Anspruch 1, wobei das distale Ende (128) von der Ummantelung (122) mit einem Winkel in dem Bereich von 15 bis 165 Grad vorgekrümmt ist.

5. Vorrichtung nach Anspruch 4, wobei das distale Ende (128) von der Ummantelung (122) mit einem Winkel in dem Bereich von 80 bis 165 Grad vorgekrümmt ist.

6. Vorrichtung nach Anspruch 1, wobei das distale Ende (130) des Mandrins (124) mit einer primären Krümmung und einer sekundären Krümmung vorgekrümmt ist.

7. Vorrichtung nach Anspruch 6, wobei die primäre Krümmung eine Bogenform von 180 Grad aufweist.

8. Vorrichtung nach Anspruch 6, wobei die sekundäre Krümmung proximal und anschließend zu der primären Krümmung liegt.

9. Vorrichtung nach Anspruch 6, wobei die sekundäre Krümmung einen größeren Krümmungsradius als die primäre Krümmung aufweist.

10. Vorrichtung nach Anspruch 6, wobei die primäre Krümmung und/oder die sekundäre Krümmung zusammengesetzte Krümmungen sind.

11. Vorrichtung nach Anspruch 1, wobei die Leitung (100) eine geschlossenendige distale Spitze (106) mit einer Form aufweist, welche dem Vorschieben der Ummantelung (122) über die distale Spitze einen Widerstand entgegensetzt.

12. Vorrichtung nach Anspruch 11, wobei die Form eine Kugelform umfasst.

13. Vorrichtung nach Anspruch 11, wobei die distale Spitze (106) von der Leitung (100) eine atraumatische Abdeckung für das distale Ende der Ummantelung (122) bereitstellt.

14. Vorrichtung nach Anspruch 1, wobei die Ummantelung (122) eine Steifigkeit aufweist, die ein Vorschieben entlang eines Epiduralraums der Wirbelsäule zu einer Position ermöglicht, wobei das vorgekrümmte distale Ende (128) der Ummantelung die Leitung in Richtung der Nervenwurzel führen kann.

15. Vorrichtung nach Anspruch 1, wobei die Ummantelung (122) eine Mindeststeifigkeit von 4,48 kPa (0,65 lbs/in²) aufweist.

16. Vorrichtung nach Anspruch 1, wobei die Ummantelung (122) aus einem Material besteht, das ausgewählt ist aus Polyimid und Polyetheretherketon.

17. Vorrichtung nach Anspruch 1, das ferner eine zusätzliche Ummantelung (122') mit einem distalen Ende aufweist, wobei die zusätzliche Ummantelung konfiguriert ist, dass sie durch die Ummantelung (122) derart hindurchgeht, dass ihr distales Ende sich über das gekrümmte distale Ende (128) der Ummantelung hinaus erstreckt.

18. Vorrichtung nach Anspruch 17, wobei das distale Ende der zusätzlichen Ummantelung (122') derart gekrümmt ist, dass die Positionierung des vorgekrümmten distalen Endes (128) der Ummantelung (122) über der Leitung (100) die Leitung entlang einer ersten Krümmung biegt und wobei das Vorschieben des gekrümmten distalen Endes von der zusätzlichen Ummantelung über das vorgekrümmte distale Ende der Ummantelung hinaus die Leitung entlang einer zweiten Krümmung biegt.

19. Vorrichtung nach Anspruch 17, wobei das distale Ende der zusätzlichen Ummantelung (122') im Wesentlichen gerade ist, sodass die Positionierung des vorgekrümmten distalen Endes (128) der Ummantelung (122) über der Leitung (100) die Leitung entlang einer ersten Krümmung biegt und wobei das Vorschieben des distalen Endes von der zusätzlichen Ummantelung über das vorgekrümmte distale Ende der Ummantelung hinaus die Leitung in einer im Wesentlichen geraden Ausrichtung führt.

20. Vorrichtung nach Anspruch 1, wobei die Leitung (100) so dimensioniert ist, dass sie einen Innendurchmesser der Ummantelung (122) derart ausfüllt, dass sie einem Abknicken der Ummantelung entgegenwirkt.

21. Vorrichtung nach Anspruch 1, wobei die Ummantelung (122) aus einem duroplastischen Material besteht.

22. Vorrichtung nach Anspruch 1, wobei die Ummantelung (122) aus einem Material mit einheitlicher Durometerhärte besteht.

23. Vorrichtung nach Anspruch 1, wobei die Ummantelung (122) Steifigkeitsübergänge aufweist.

24. Vorrichtung nach Anspruch 1, wobei die Ummantelung (122) aus einer Metall/Polymer-Konstruktion besteht.

25. Vorrichtung nach Anspruch 1, wobei die Ummantelung (122) mindestens teilweise strahlenundurchlässig ist.

26. Vorrichtung nach Anspruch 25, wobei die Ummantelung (122) mit Röntgenkontrastmittel beaufschlagt ist oder wobei die Ummantelung mindestens eine röntgendichte Markierung enthält.

## Revendications

1. Système pour avoir accès à une racine nerveuse qui s'étend à partir d'une colonne vertébrale le long d'une angulation de manchon de racine nerveuse, comprenant:
un fil (100) comprenant une tige (103) ayant une lumière de stylet (104) s'étendant au moins partiellement à travers cette dernière et au moins une électrode (102) disposée sur cette dernière;
une gaine (122) ayant une extrémité distale pré-incurvée (128), dans lequel la gaine est configurée pour s'étendre sur la tige du fil; et
un stylet (124) ayant une extrémité distale pré-incurvée (130), dans lequel le stylet est configuré pour être positionné à l'intérieur de la lumière de stylet du fil,
dans lequel le fil est configuré pour être positionné le long de la colonne vertébrale, dans lequel le positionnement de l'extrémité distale pré-incurvée de la gaine sur le fil courbe le fil le long d'une première courbure et dans lequel l'avancement du fil et du stylet à l'intérieur de ce dernier au-delà de la gaine courbe le fil le long d'une seconde courbure.

2. Système selon la revendication 1, dans lequel la gaine (122) est configurée pour être avancée par le biais d'une aiguille d'introduction (126) configurée pour avoir accès à un espace épidural de la colonne vertébrale.

3. Système selon la revendication 2, dans lequel l'aiguille d'introduction (126) a un diamètre interne inférieur ou égal à 1,7 mm (0,067 pouces).

4. Système selon la revendication 1, dans lequel l'extrémité distale (128) de la gaine (122) est pré-incurvée avec un angle de l'ordre de 15 à 165 degrés.

5. Système selon la revendication 4, dans lequel l'extrémité distale (128) de la gaine (122) est pré-incurvée avec un angle de l'ordre de 80 à 165 degrés.

6. Système selon la revendication 1, dans lequel l'extrémité distale (130) du stylet (124) est pré-incurvée avec une courbe principale et une courbe secondaire.

7. Système selon la revendication 6, dans lequel la courbe principale a une forme d'arc de 180 degrés.

8. Système selon la revendication 6, dans lequel la courbe secondaire est proximale et adjacente à la courbe principale.

9. Système selon la revendication 6, dans lequel la courbe secondaire a un plus grand rayon de courbure que la courbe principale.

10. Système selon la revendication 6, dans lequel la courbe principale et/ou la courbe secondaire sont des courbes composées.

11. Système selon la revendication 1, dans lequel le fil (100) a une pointe distale à extrémité fermée (106) ayant une forme qui résiste à l'avancement de la gaine (122) sur la pointe distale.

12. Système selon la revendication 11, dans lequel la forme comprend une forme de bille.

13. Système selon la revendication 11, dans lequel la pointe distale (106) du fil (100) fournit un couvercle atraumatique pour l'extrémité distale de la gaine (122).

14. Système selon la revendication 1, dans lequel la gaine (122) a une rigidité qui permet l'avancement le long d'un espace épidural de la colonne vertébrale jusqu'à une position dans laquelle l'extrémité distale pré-incurvée (128) de la gaine peut diriger le fil vers la racine nerveuse.

15. Système selon la revendication 1, dans lequel la gaine (122) a une rigidité minimum de 4,48 kPa (0,65 livres. pouce²).

16. Système selon la revendication 1, dans lequel la gaine (122) est composée d'un matériau choisi parmi le polyimide et le polyétheréthercétone.

17. Système selon la revendication 1, comprenant en outre une gaine supplémentaire (122') ayant une extrémité distale, dans lequel la gaine supplémentaire est configurée pour passer à l'intérieur de la gaine (122) de sorte que son extrémité distale s'étend au-delà de l'extrémité distale incurvée (128) de la gaine.

18. Système selon la revendication 17, dans lequel l'extrémité distale de la gaine supplémentaire (122') est incurvée de sorte que le positionnement de l'extrémité distale pré-incurvée (128) de la gaine (122) sur le fil (100) courbe le fil le long de la première courbure et dans lequel l'avancement de l'extrémité distale incurvée de la gaine supplémentaire au-delà de l'extrémité distale pré-incurvée de la gaine courbe le fil le long de la seconde courbure.

19. Système selon la revendication 17, dans lequel l'extrémité distale de la gaine supplémentaire (122') est sensiblement droite de sorte que le positionnement de l'extrémité distale pré-incurvée (128) de la gaine (122) sur le fil (100) courbe le fil le long de la première courbure et dans lequel l'avancement de l'extrémité distale de la gaine supplémentaire au-delà de l'extrémité distale pré-incurvée de la gaine dirige le fil dans une direction sensiblement droite.

20. Système selon la revendication 1, dans lequel le fil (100) est dimensionné pour remplir un diamètre interne de la gaine (122) afin de résister à la torsion de la gaine.

21. Système selon la revendication 1, dans lequel la gaine (122) est composée d'une matière thermodurcie.

22. Système selon la revendication 1, dans lequel la gaine (122) est composée d'une matière à duromètre unique.

23. Système selon la revendication 1, dans lequel la gaine (122) présente des transitions de rigidité.

24. Système selon la revendication 1, dans lequel la gaine (122) est composée d'une structure en métal/polymère.

25. Système selon la revendication 1, dans lequel la gaine (122) est au moins partiellement radio-opaque.

26. Système selon la revendication 25, dans lequel la gaine (122) est chargée avec une matière radio-opaque ou bien dans lequel la gaine comprend au moins un marqueur radio-opaque.
